(19) Europäisches Patentamt
European Patent Office
Office européen des brevets

(11) Publication number : **0 658 906 A2**

(12) **EUROPEAN PATENT APPLICATION**

(21) Application number : **94810713.1**

(51) Int. Cl.[6] : **H01B 1/12,** C12N 11/08

(22) Date of filing : **09.12.94**

(30) Priority : **18.12.93 GB 9325946**

(43) Date of publication of application :
**21.06.95 Bulletin 95/25**

(84) Designated Contracting States :
**BE CH DE ES FR GB IT LI NL SE**

(71) Applicant : **Japat Ltd**
**Klybeckstrasse 141**
**CH-4057 Basel (CH)**

(72) Inventor : **Wernet, Wolfgang, Dr.**
**3-14-12-301, Shinoharakitamachi,**
**Nada-ku**
**Kobe-shi, Hyogo-ken 657 (JP)**
Inventor : **Khan, Golam F., Dr.**
**20-302, Igazuka,**
**Nishihirano,**
**Mikage-cho**
**Higashinada-ku, Kobe-shi, Hyogo 658 (JP)**

(74) Representative : **Dannappel, Hans-Jochen et al**
**c/o CIBA-GEIGY AG**
**Patentabteilung**
**Postfach**
**CH-4002 Basel (CH)**

(54) **Protein adsorbed shapable electroconductive polymer film.**

(57) A protein adsorbed shapable electroconductive polymer film comprising
1) a film made of a composition comprising
a) at least one polyheteroaromatic compound or aniline in oxidized, polycationic form, and
b) at least one polyanion of a film-forming thermoplastic polymer containing sulfated alcoholic groups

$$-\overset{|}{\underset{|}{C}}-O-SO_3^{\ominus}$$

and/or sulfonatalkylated groups

$$-\overset{|}{\underset{|}{C}}-O-(C_nH_{2n})-SO_3^{\ominus}$$

in repeating structural units, wherein the group $-(C_nH_{2n})-$ is linear or branched $C_2$-$C_{12}$-alkylene containing 2 to 5 C-atoms in the carbon main chain, the alkylene being unsubstituted or substituted by $C_1$-$C_4$-alkoxy ; and
2) a protein adsorbed on said film.
This protein adsorbed shapable electroconductive polymer film can be suitably applied as biosensors, bioreactor materials and immunosensors.

EP 0 658 906 A2

The present invention relates to a novel protein adsorbed shapable electroconductive polymer film to be suitably used as sensing electrodes of biosensors, sensing electrodes of immunosensors, materials for bioreactors and others.

Since the development of electroconductive polymers, polypyrrole, especially, has been recognized as an excellent material for biosensors in which a desired protein such as glucose oxidase is incorporated as an element to detect a substrate to be determined (see, for example, L.D.Couves et al., Synthetic Metals, 28(1989)C761-C768, W.Schuhmann et al., Biosensors & Bioelectronics, 6(1991)689-697, G.Zotti, Synthetic Metals, 51(1992)373-382, G.Bidan, Sensors and Actuator B, 6(1992)45-56 and M.Aizawa, Analytica Chimica Acta, 250(1991)249-256).

In a typical application of polypyrrole to biosensors, pyrrole monomers are electrolytically polymerized in the presence of a desired protein such as glucose oxidase or the like and if necessary, some other components like mediators or promotors, onto the surface of a suitable electroconductive support, whereby the protein is immobilized (together with the mediators or promotors, if added) in the formed polypyrrole film, and serves as a sensor with extremely high specificity when immersed in a solution containing a substance to be determined (see, for example, M.Aizawa, Analytica Chimica Acta, 250(1991)259-256, G. Zotti, Synthetic Metals, 51(1992)373-382, W.Schuhmann et al., Biosensors & Bioelectronics, 6(1991)689-697 and L.D.Couves et al., Synthetic Metals, 28(1989)C761-C768).

As another application of electroconductive polymers, it is proposed to apply an electroconductive polymer such as polypyrrole to immunosensors with high sensitivity which take advantage of the change in surface potential of a polypyrrole film to which an antigen is immobilized by electrochemical co-deposition, the change being caused by antigen-antibody reaction (see, for example, H.Uchida et al., Sensors and Materials, 2, 3(1990)137-146).

However, in spite of the excellent characteristics and performance of the above biosensors, immunosensors and bioreacter materials, they have the following drawbacks:

- a desired protein (and mediators or promoters, if necessary) must be immobilized during the electrolytic polymerization process of polypyrrole, which often leads to denaturation of the protein, resulting in the absence of the desired performance,
- the fabrication process is complicated and expensive,
- they are no more shapable after preparation because of the presence of the support, limiting the scope of application,
- their long term stability is poor since they are based on conventional polypyrrole films doped with small anions, and
- the electroconductive polymer films as active layers are often rigid and unshapable or brittle in themselves.

It has now been found, surprisingly, that an electroconductive polymer film doped with specific polymeric anions with high stability, high flexibility and high shapability unexpectedly shows strong hydrophobic interaction with proteins, and that a protein can be immobilized on the surface of the above electroconductive polymer film by simply immersing the film in an aqueous solution containing the protein without using any electrolytic process, thereby simplifying the production process of biosensors, immunosensors, bioreactor materials and others, eliminating the danger of the denaturation of the immobilized proteins, and yielding biosensors, immunosensors, bioreactor materials and others with high stability, high flexibility and high shapability.

The present invention relates, therefore, to a protein adsorbed shapable electroconductive polymer film comprising

1) a film made of a composition comprising

a) at least one polyheteroaromatic compound or aniline in oxidized, polycationic form, and

b) at least one polyanion of a film-forming thermoplastic polymer containing sulfated alcoholic groups

$$-\overset{|}{\underset{|}{C}}-O-SO_3^{\ominus}$$

and/or sulfonatalkylated groups

$$-\overset{|}{\underset{|}{C}}-O-(C_nH_{2n})-SO_3^{\ominus}$$

in repeating structural units, wherein the group $-(C_n,H_{2n})-$ is linear or branched $C_2$-$C_{12}$-alkylene containing 2 to 5 C-atoms in the carbon main chain, the alkylene being unsubstituted or substituted by $C_1$-$C_4$-alkoxy; and

2) a protein adsorbed on the surface of said film.

Polyheteroaromatic compounds will be understood within the scope of this invention as meaning homopolymers and copolymers which contain repeating heteroaromatic structural units. They may be high molecular or also oligomeric, with the proviso that they are solid at room temperature and are able to form films. Preferred polyheteroaromatic compounds are those of 5-membered rings which contain 1 to 3 hetero atoms, preferably 1 hetero atom, selected from the group consisting of -O-, -S- and -N-, and carbon atoms which are unsubstituted or substituted by $C_1$-$C_{18}$-alkyl, preferably by $C_1$-$C_{12}$-alkyl. Preferably two carbon atoms are not substituted, so as to be able to carry out the electrochemical polymerization. The 5-membered ring is preferably selected from the group consisting of pyrrole, thiophene, furan, 2,2'-bipyrrole, 2,2'-bithiophene, 2,2'-bifuran, thiazole, oxazole, thiadiazole and imidazole.

Especially preferred are the polyheteroaromatic compounds of a pyrrole of formula (I)

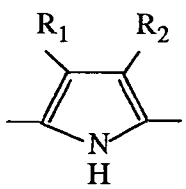

(I),

wherein $R_1$ and $R_2$ are each independently of the other hydrogen or $C_1$-$C_{16}$-alkyl. $R_1$ and $R_2$ may be $C_1$-$C_{12}$-alkyl, for example, methyl or ethyl, and are preferably hydrogen. The NH-group of the pyrrole may be substituted by $C_1$-$C_{12}$-alkyl, preferably $C_1$-$C_6$-alkyl; and the polyheteroaromatic compounds of a thiophene of formula (II)

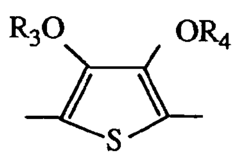

(II),

wherein $R_3$ and $R_4$ are each independently of the other linear or branched $C_1$-$C_{18}$-alkyl or $C_2$-$C_{18}$-alkoxyalkyl; or $C_3$-$C_8$-cycloalkyl, phenyl or benzyl which may be unsubstituted or substituted by $C_1$-$C_6$-alkyl, $C_1$-$C_6$-alkoxy or halogen; or $R_3$ and $R_4$ together represent linear $C_1$-$C_6$-alkylene which is unsubstituted or substituted by $C_1$-$C_6$-alkyl, $C_1$-$C_6$-alkoxy, halogen, $C_3$-$C_8$-cycloalkyl, phenyl, benzyl, $C_1$-$C_4$-alkylphenyl, $C_1$-$C_4$-alkoxyphenyl, halogenphenyl, $C_1$-$C_4$-alkylbenzyl, $C_1$-$C_4$-alkoxybenzyl or halogenbenzyl.

$R_3$ and $R_4$ as alkyl represent preferably $C_1$-$C_{12}$-alkyl, more preferably $C_1$-$C_8$-alkyl, and especially $C_1$-$C_4$-alkyl. Examples of such alkyl are methyl, ethyl and the isomers of propyl, butyl, pentyl, hexyl, heptyl, octyl, nonyl, decyl, undecyl, dodecyl, tetradecyl, hexadecyl and octadecyl. Preferred alkyl residues are methyl, ethyl, n- and i-propyl, n-, i- and t-butyl, and further n-pentyl and n-hexyl.

$R_3$ and $R_4$ as alkoxyalkyl represent preferably $C_1$-$C_{17}$-alkoxymethyl or $C_1$-$C_{16}$-alkoxyethyl, preferably $C_1$-$C_{12}$-alkoxymethyl or $C_1$-$C_{12}$-alkoxyethyl, more preferably $C_1$-$C_8$-alkoxymethyl or $C_1$-$C_8$-alkoxyethyl, and especially $C_1$-$C_4$-alkoxymethyl or $C_1$-$C_4$-alkoxyethyl. Examples of such alkoxyalkyl are methoxymethyl, ethoxymethyl, n- or i-propyloxymethyl, n-, i- or t-butyloxymethyl, methoxyethyl, ethoxyethyl, n- or i-propyloxyethyl, n-, i- and t-butyloxyethyl.

If $R_3$ and $R_4$ represent cycloalkyl, then $C_5$- or $C_6$-cycloalkyl such as cyclopentyl and cyclohexyl are preferred.

As the substituents for cycloalkyl, phenyl and benzyl, $C_1$-$C_4$-alkyl, $C_1$-$C_4$-alkoxy, F, Cl and Br are preferred. Examples of such alkyl and alkoxy are, especially, methyl, ethyl, methoxy and ethoxy. Examples of such substituted residues are methylcyclopentyl, methylcyclohexyl, methoxycyclohexyl, methylphenyl, dimethylphenyl, ethylphenyl, methoxyphenyl, dimethoxyphenyl, chlorophenyl, methylbenzyl, dimethylbenzyl, ethylbenzyl, methoxybenzyl, dimethoxybenzyl and chlorobenzyl.

If $R_3$ and $R_4$ together represent alkylene, then $C_1$-$C_4$-alkylene, $C_1$- or $C_2$-alkylene in particular, are preferred. Examples of such alkylene are methylene, ethylene, propylene, butylene, pentylene and hexylene.

Preferred substituents for $R_3$ and $R_4$ as alkylene are $C_1$-$C_6$-alkyl such as methyl, ethyl propyl and butyl, $C_1$-$C_6$-alkoxy such as methoxy and ethoxy, cyclohexyl, phenyl and benzyl.

In a preferred subgroup, $R_3$ and $R_4$ mean linear or branched $C_1$-$C_6$-alkyl, especially $C_1$-$C_4$-alkyl, or $R_3$ and $R_4$ together mean $C_1$-$C_4$-alkylene, especially $C_1$- or $C_2$-alkylene which is unsubstituted or substituted by 1 or

2 $C_1$-$C_6$-alkyl groups or $C_1$-$C_6$-alkoxy groups.

In a preferred embodiment, $R_3$ and $R_4$ are $C_1$- or $C_2$-alkylene which is unsubstituted or substituted by $C_1$-$C_6$-alkyl or $C_1$-$C_6$-alkoxy.

In a more preferred embodiment, $R_3$ and $R_4$ represent a residue of formula $-CHR_a-CHR_b-$, wherein $R_a$ and $R_b$ are each independently of the other H or $C_1$-$C_6$-alkyl, especially methyl or ethyl.

In an especially preferred embodiment, the structural unit of formula (II) corresponds to the residue of formula

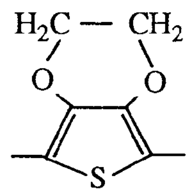 .

Anilines may be aniline itself or anilines which are substituted in 3-position by $C_1$-$C_{12}$-alkyl, preferably $C_1$-$C_6$-alkyl.

The composition of this invention contains in each structural unit of the polyheteroaromatic compound preferably 0.1 to 0.9, more preferably 0.1 to 0.6, most preferably 0.15 to 0.4, structural units containing sulfated alcoholic groups

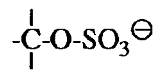

and/or sulfonatalkylated alcoholic groups

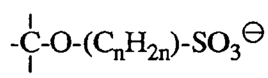 .

The group $-(C_nH_{2n})-$ preferably represents linear or branched $C_3$-$C_8$-alkylene having 3 to 5 C-atoms in the carbon chain. Particularly preferred are linear $C_3$-$C_8$-alkylene. Examples of such alkylene are ethylene, methylethylene, dimethylethylene, 1,3-propylene, methyl-1,3-propylene, dimethyl-1,3-propylene, trimethyl-1,3-propylene, 1,4-butylene, methyl-1,4-butylene, dimethyl-1,4-butylene, trimethyl-1,4-butylene, tetramethyl-1,4-butylene, 1,3- or 1,5-pentylene and 1,3-, 1,4-, 1,5- or 1,6-hexylene. In particular, trimethylene and tetramethylene are preferred as the group $-(C_nH_{2n})-$.

The thermoplastic polymers eligible for use in the composition of this invention containing sulfated alcoholic groups

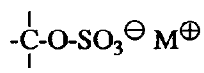

and/or sulfonatalkylated alcoholic groups

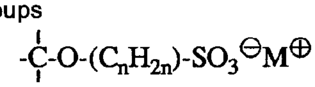

in salt form preferably have a glass transition temperature of -100° to 350°C, more preferably -50° to 250°C, measured by DSC method (Differential-Scanning-Calorimetry). M means an alkali metal- or ammonium cation, which are hereinafter described in more detail.

The tensile strength of these thermoplastic polymers is preferably not less than 5 MPa, more preferably not less than 10 MPa, determined according to DIN 53 455. Depending on the nature of the polymer, the tensile strength can be up to 1000 MPa, preferably up to 500 MPa, and most preferably up to 300 MPa. The polymerization degree can be, for example, 5 to 10000, preferably 10 to 5000, and most preferably 10 to 1000.

The alcoholic groups in the thermoplastic polymer can be sulfated and/or sulfonatalkylated partially or completely. The ratio of free alcoholic groups to sulfated alcoholic groups

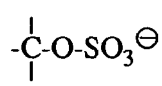

and/or sulfonatalkylated alcoholic groups

$$-\overset{|}{\underset{|}{C}}-O-(C_nH_{2n})-SO_3^{\ominus}$$

in the thermoplastic polymer can be, for example, from 50:1 to 1:50, preferably from 10:1 to 1:10.

In a preferred embodiment, the thermoplastic polymer contains 5 to 100 mol%, preferably 10 to 100 mol%, more preferably 20 to 100 mol%, especially 30 to 100 mol%, and most preferably 30 to 90 mol%, of sulfated alcoholic groups

$$-\overset{|}{\underset{|}{C}}-O-SO_3^{\ominus}$$

and/or sulfonatalkylated alcoholic groups

$$-\overset{|}{\underset{|}{C}}-O-(C_nH_{2n})-SO_3^{\ominus},$$

wherein the remaining structural units are corresponding hydroxy group-containing or hydroxyl group-free structural units.

The sulfated and/or sulfonatalkylated alcoholic groups can be bound as secondary $>CH-O-SO_3^{\ominus}$ or $>CH-O-(C_nH_{2n})SO_3^{\ominus}$ groups or as tertiary

$$-\overset{|}{\underset{|}{C}}-O-SO_3^{\ominus}$$

or

$$-\overset{|}{\underset{|}{C}}-O-(C_nH_{2n})-SO_3^{\ominus}$$

groups in the polymer backbone; or in side-chains of the polymer as primary $-CH_2-O-SO_3^{\ominus}$ or $-CH_2-O-(C_nH_{2n})SO_3^{\ominus}$ groups in the terminal position, or as secondary $>CH-O-SO_3^{\ominus}$ or $>CH-O-(C_nH_{2n})SO_3^{\ominus}$ or as tertiary

$$-\overset{|}{\underset{|}{C}}-O-SO_3^{\ominus}$$

or

$$-\overset{|}{\underset{|}{C}}-O-(C_nH_{2n})-SO_3^{\ominus}$$

in a central position of the side chain.

The thermoplastic polymers may be derived from polymers or mixtures thereof containing different hydroxyl groups, for example polyesters, polyesteramides, polyurethanes, polyimides, polycarbonates and polyimides obtained from hydroxyl-containing monomers; saponified homopolymers of vinyl esters or ethers; hydroxylated polyolefines such as hydroxylated polybutadiene, polyisoprene or chloroprene as well as copolymers thereof with olefinic monomers; homopolyacrylates or homopolymethacrylates containing hydroxyalkyl groups in the ester moiety; polysiloxanes containing hydroxyalkyl groups; reduced polyketones or copolymers thereof; polymers obtained from glycidyl compounds and diols; polyvinylphenols or copolymers of vinylphenols and olefinic comonomers; as well as copolymers of vinyl alcohol, acrylates or methacrylates containing hydroxyalkyl groups, or hydroxylated diolefines with ethylenic unsaturated comonomers such as acrylonitrile, olefines, diolefines, vinylchloride, vinyliden chloride, vinyl fluoride, vinyliden fluoride, styrene, α-methylstyrene, maleic anhydride, maleic acid imide, vinylethers and vinylesters.

The sulfated and/or sulfonatalkylated thermoplastic polymers are preferably derived from polymers selected from the group consisting of polyadducts of glycidyl compounds containing on average more than one epoxy group with a diol; homo- and copolymers of hydroxyalkyl acryrlates and methacrylates; homo- and copolymers of butadiene, isoprene and chloroprene, the double bonds thereof being partially or completely hydroxylated; homoand copolymers of vinylphenols; polyimides of hydrogenated ketotetracarboxylic acids, especially benzophenonetetracarboxylic acid; hydroxyalkyl polysiloxanes; and polyesters, polyamides, polyurethanes and polyimides of $C_4$-$C_{12}$-alkenylenediols or alkenylenediamines, the double bond of which are hydroxylated.

The thermoplastic polymer preferably contains either sulfated or sulfonatalkylated hydroxyl groups.

The thermoplastic polymer may be, for example, an at least partially sulfated and/or sulfonatalkylated polyadduct of

a) a glycidyl compound containing on average more than one epoxy group and

b) a diol which contains

$$\begin{array}{l}-CH-\\ \ \ |\\ \ \ OSO_3^{\ominus}\end{array}$$

and/or

$$\begin{array}{l}-CH-\\ \ \ |\\ \ \ O(C_nH_{2n})SO_3^{\ominus}\end{array}$$

in the polymer chain, wherein the group $-(C_nH_2)_n-$ has the meaning given hereinbefore including preferred ones.

The polyadducts are preferably derived from glycidyl compounds containing on average two epoxy groups in the molecule.

Particularly suitable glycidyl compounds are those containing two glycidyl, β-methylglycidyl or 2,3-epoxycyclopentyl groups attached to a hetero atom (for example, a sulfur atom, preferably an oxygen or a nitrogen atom). Such compounds are in particular: bis(2,3-epoxycyclopentyl)ethers; diglycidyl ethers of polyhydric aliphatic alcohols such as 1,4-butanediol, or polyalkylene glycols such as polypropylene glycols; diglycidyl ethers of cycloaliphatic polyols such as 2,2-bis(4-hydroxycyclohexyl)propane; diglycidyl ethers of polyhydric phenols such as resorcinol, bis(hydroxyphenyl)methane, 2,2-bis(p-hydroxyphenyl)propane (diomethane), 2,2-bis(4'-hydroxy-3,5'-dibromophenyl)propane, 1,3-bis(p-hydroxyphenyl)ethane; bis(β-methylglycidyl)ethers of the above mentioned dihydric alcohols or dihydric phenols; diglycidyl esters of dicarboxylic acids such as phthalic acid, terephthalic acid, $\Delta^4$-tetrahydrophthalic acid and hexahydrophthalic acid; N,N-diglycidyl derivatives of primary amines and amides and heterocyclic nitrogen bases containing two nitrogen atoms, and N,N'-diglycidyl derivatives of di-secondary diamides and diamines such as N,N-diglycidylaniline, N,N-diglycidyltoluidine, N,N-diglycidyl-p-aminophenylmethyl ether, N,N'-dimethyl-N,N'-diglycidyl-bis(p-aminophenyl)methane; N',N"-diglycidyl-N-phenylisocyanurate; N,N'-diglycidylethyleneurea; N,N'-diglycidyl-5,5-dimethylhydantoin, N,N'-diglycidyl-5-isopropylhydantoin, N,N-methylene-bis(N',N-diglycidyl-5,5-dimethylhydantoin), 1,3-bis(N-glycidyl-5,5-dimethylhydantoin)-2-hydroxypropane; N,N'-diglycidyl-5,5-dimethyl-6-isopropyl-5,6-dihydrouracil.

The glycidyl compounds can be reacted with aliphatic, cycloaliphatic or aromatic diols to the preferred polyadducts, in which reaction a secondary alcohol group is formed at the glycidyl group which can be sulfated or sulfonatalkylated.

The glycidyl compounds can also be reacted with primary aliphatic, cycloaliphatic or aromatic monoamines (for example, aniline, toluidine, $C_1$-$C_{12}$-alkylamines, $C_2$-$C_{12}$-hydroxyalkylamines), aliphatic, cycloaliphatic or aromatic dicarboxylic acids (for example, maleic acid, adipic acid, tetramethyladipic acid, sebacic acid, azelaic acid, succinic acid, dodecylsuccinic acid, phthalic acid, terephthalic acid, $\Delta^4$-tetrahydrophthalic acid, hexahydrophthalic acid, 4-methylhexahydrophthalic acid, 3,6-endomethylene-$\Delta^4$-tetrahydrophthalic acid, 4-methyl-3,6-endomethylene-$\Delta^4$-tetrahydrophthalic acid), or with aliphatic, cycloaliphatic, heterocyclic or aromatic di-secondary amines or di-secondary carboxamides (for example, N,N'-dimethylethylenediamine, N,N'-dimethylpropylene-1,3-diamine, N,N'-dimethylhexamethylenediamine, N,N'-dicyclohexylhexamethylenediamine, N,N',N"-trimethyldiethylenetriamine, N,N'-diethylpropylene-1,3-diamine, N-methyl-3,5,5-trimethyl-3-(methylaminomethyl)cyclohexylamine, N,N'-dimethylated or N,N'-diethylated aromatic diamines, for example, m- or

p-phenylenediamine, bis(4-aminophenyl)methane or -sulfone, 2,2-bis(4-aminophenyl)propane, N,N-dimethyl-m-xylenediamine, as well as ethyleneurea, 5,5-dimethylhydantoin, 5-isopropylhydantoin, N,N-methylenebis-5,5-dimethylhydantoin, 1,3-bis(5,5-dimethyl)-2-hydroxypropane, 5,5-dimethyl-6-isopropyl-5,6-dihydrouracil), by polyaddition to give linear polyadducts.

A preferred composition to be used in this invention is one in which the polyadduct contains

a) 100 to 5 mol% of identical or different structural units of formula III and/or IIIa

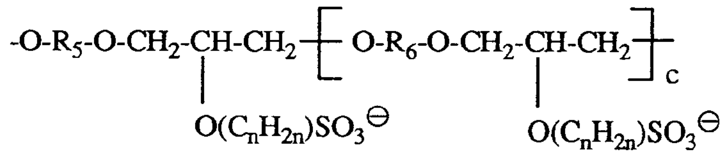

(III)

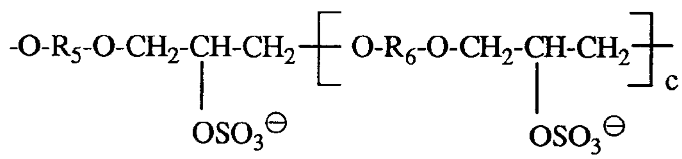

(IIIa)

and

b) 0 to 95 mol% of identical or different structural units of formula IV

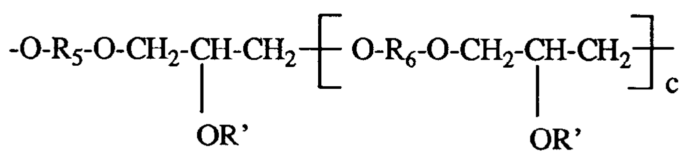

(IV)

based on said adduct, in which formulae (III), (IIIa) and (IV) $R_5$ and $R_5$ are each independently of the other the residue of a diol which contains aliphatic or aromatic diol groups, which residue is diminished by two hydroxyl groups, R' is hydrogen, $C_1$-$C_{20}$-alkyl, $C_1$-$C_{20}$-acyl or aminocarbonyl which is N-substituted by a $C_1$-$C_{20}$-hydrocarbon group, the group $-(C_nH_2)_n-$ has the meaning as given hereinbefore including preferred ones, and c is 0 or 1.

R' as $C_1$-$C_{20}$-alkyl can be linear or branched. R' as acyl can be, for example, $C_1$-$C_{20}$-alkyl-CO-, $C_5$-$C_8$-cycloalkyl-CO-, $C_1$-$C_{15}$-alkyl-$C_5$-$C_8$-cycloalkyl-CO-, $C_5$-$C_8$-cycloalkyl-$CH_2$-CO-, $C_1$-$C_{14}$-alkyl-$C_5$-$C_8$-cycloalkyl-$CH_2$-CO-, phenyl-CO-, benzyl-CO-, $C_1$-$C_{14}$-alkylphenyl-CO- or -benzyl-CO-. The hydrocarbon group in aminocarbonyl can be, for example, $C_1$-$C_{20}$-alkyl-, $C_5$-$C_8$-cycloalkyl, $C_1$-$C_{15}$-alkyl-$C_5$-$C_8$-cycloalkyl-, $C_5$-$C_8$-cycloalkyl-$CH_2$-, $C_1$-$C_{14}$-alkyl-$C_5$-$C_8$-cycloalkyl-$CH_2$-, phenyl-, benzyl-, $C_1$-$C_{15}$-alkylphenyl- or -benzyl-. R' preferably represents hydrogen, $C_1$-$C_6$-acyl or $C_1$-$C_4$-alkyl. R' is most preferably hydrogen.

The polyadduct contains preferably 100 to 20 mol%, more preferably 100 to 30 mol%, of structural units of formula III and/or IIIa, and preferably 0 to 80 mol%, more preferably 0 to 70 mol%, of structural units of formula IV. Most preferably, the polyadduct contains 90 to 60 mol% of structural units of formula III and/or IIIa and 0 to 40 mol% of structural units of formula IV.

In a preferred embodiment of the invention, $R_5$ and $R_6$ are identical residues. $R_5$ and $R_6$ as a residue having aliphatic diol groups contain preferably 2 to 12, most preferably 2 to 8, carbon atoms. The hydroxyl groups can be attached to an open-chain or cyclic aliphatic residue. A suitable aliphatic residue is, for example, linear or branched $C_2$-$C_{12}$-alkylene, $C_3$-$C_8$-cycloalkylene, $C_1$-$C_4$-alkyl-$C_5$-$C_8$-cycloalkylene, cyclohexylmethylene or cyclohexyldimethylene. Examples of such aliphatic residues are ethylene, 1,2- or 1,3-propylene, 1,2-, 1,3- or 1,4-butylene, 1,2-, 1,3-, 1,4- or 1,5-pentylene, 1,2-, 1,3-, 1,4-, 1,5- or 1,6-hexylene, heptylene, octylene, nonylene, decylene, undecylene, dodecylene, 1,3-cyclopentylene, 1,3- or 1,4-cyclohexylene, 2-methyl-1,4-cyclohexylene and cyclohexyl-1,4-dimethylene.

The aromatic diol groups of the diols to be used for the polyadducts are preferably phenolic groups. The diol residues carrying phenolic groups contain preferably 6 to 30, most preferably 6 to 20, carbon atoms. A preferred embodiment of the invention relates to compositions wherein $R_5$ and $R_6$ are each independently of the

other a residue of formula

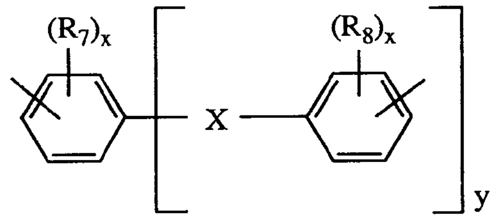

(V),

wherein X means a direct bond, $C_1$-$C_4$-alkylene, $C_2$-$C_{12}$-alkylidene, $C_5$-$C_8$-cycloalkylidene, -O-, -S-, $-SO_2-$, -CO-, $-CO_2-$, -N($C_1$-$C_4$-alkyl)- or $-Si(CH_3)_2-$, $R_7$ and $R_8$ are each independently of the other hydrogen, halogen, $C_1$-$C_4$-alkyl or $C_1$-$C_4$-alkoxy, and x means 1 or 2 and y is 0 or 1.

X is preferably a direct bond, methylene, ethylene, $C_2$-$C_6$-alkylidene, cyclohexylidene or cyclopentylidene, -O-, or -S-. $R_7$ and $R_8$ are preferably hydrogen and y is preferably 1.

$R_5$ and $R_6$ especially mean the residue

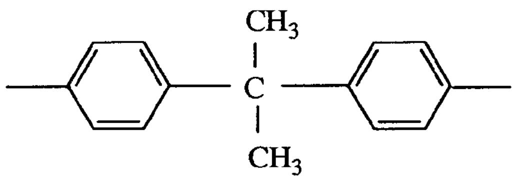

.

Another preferred composition to be used in the present invention is one wherein the thermoplastic polymer is a polyvinyl alcohol which is at least partially sulfated and/or sulfonatalkylated or a sulfated and/or sulfonatalkylated polyvinyl alcohol-copolymer having the groups of

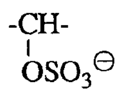

and/or

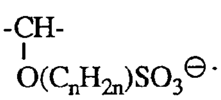

Sulfated and/or sulfonatalkylated polyvinyl alcohol-copolymers are preferably contained in the composition.

Preferred are compositions with polyvinyl alcohol-copolymers, wherein the copolymer contain

a) 90 to 5 mol% of structural units of formula VI and/or VIa

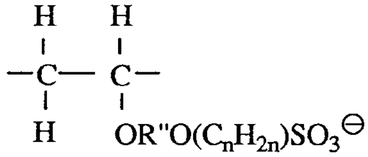

(VI)

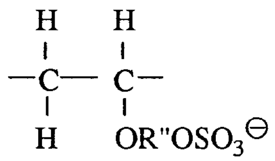

(VIa)

and

b) 10 to 95 mol% of identical or different structural units of formula VII

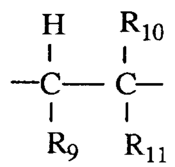

(VII)

wherein $R_9$ stands for hydrogen, $C_1$-$C_6$-alkyl, -$COOR_{12}$ or -$COO^{\ominus}$, $R_{10}$ means H, F, Cl, CN or $C_1$-$C_6$-alkyl, and $R_{11}$ represents H, OH, R"OH, F, Cl, CN, $R_{12}$-O-, $C_1$-$C_{12}$-alkyl, -$COO^{\ominus}$, -$COOR_{12}$, -OCO-$R_{12}$, methylphenyl or phenyl, in which $R_{12}$ represents $C_1$-$C_{18}$-alkyl, $C_5$-$C_7$-cycloalkyl, ($C_1$-$C_{12}$-alkyl)-$C_5$-$C_7$-cycloalkyl, phenyl, ($C_1$-$C_{12}$-alkyl)phenyl, benzyl or ($C_1$-$C_{12}$-alkyl)benzyl and R" stands for linear or branched $C_2$-$C_{18}$-alkylene, poly($C_2$-$C_6$-oxaalkylene) containing 2 to 6 oxaalkylene units, and the group -($C_nH_{2n}$)- has the meaning given hereinbefore.

Preferably 60 to 10 mol%, especially 60 to 20 mol%, of structural units of formula VI and/or VIa, and 40 to 90 mol%, especially 40 to 80 mol%, of structural elements of formula VII, are contained.

R" as alkylene contains preferably 2 to 12, more preferably 2 to 8, and most preferably 2 to 6, carbon atoms. Examples of R" are ethylene and the isomers propylene, butylene, pentylene, hexylene, heptylene, octylene, nonylene, decylene, undecylene, dodecylene, tetradecylene, hexadecylene and octadecylene. Preferred groups are ethylene, 1,2- and 1,3-propylene, 1,2-, 1,3- and 1,4-butylene, 1,2-, 1,3-, 1,4- and 1,5-pentylene and 1,2-, 1,3-, 1,4-, 1,5- and 1,6-hexylene.

R" as poly(oxaalkylene) contains preferably 2 to 4 oxaalkylene units and preferably 2 to 4, most preferably 2 or 3, carbon atoms in the alkylene moiety.

$R_9$ stands preferably for hydrogen. If $R_9$ means alkyl, then methyl or ethyl is preferred. If $R_9$ means -$COOR_{12}$, then $R_{12}$ represents preferably $C_1$-$C_{12}$-, most preferably, $C_1$-$C_6$-alkyl.

If $R_{10}$ means alkyl, then $C_1$-$C_4$-alkyl, for example, methyl, ethyl, n-propyl and n-butyl are preferred. $R_{10}$ stands preferably for H, Cl or $C_1$-$C_4$-alkyl.

If $R_{11}$ means the group $R_{12}$-O-, $R_{12}$ represents preferably $C_1$-$C_{12}$-, especially $C_1$-$C_6$-alkyl. If $R_{11}$ means alkyl, $R_{11}$ contains preferably 1 to 6, most preferablyl to 4, carbon atoms. If $R_{11}$ means the group -$COOR_{12}$, $R_{12}$ represents preferably $C_1$-$C_{12}$-, most preferably $C_1$-$C_6$-alkyl, cycloalkyl or cyclohexyl. If $R_{11}$ means the group -OCO-$R_{12}$, $R_{12}$ represents preferably $C_1$-$C_{12}$-, especially $C_1$-$C_6$-alkyl, phenyl or benzyl.

In a preferred embodiment of the invention, $R_9$ stands for H, $R_{10}$ for H, F, Cl, methyl or ethyl, and $R_{11}$ for H, OH, F, Cl, CN, $C_1$-$C_6$-alkyl, $C_1$-$C_6$-alkoxy, -COO-$C_1$-$C_6$-alkyl, -OOC-$C_1$-$C_6$-alkyl or phenyl.

Particularly preferred compositions are those wherein in formula VII $R_9$ is H, $R_{10}$ is H or methyl, and $R_{11}$ is H, OH, CN, methyl, $OCH_3$ or -$COOCH_3$.

Most preferred compositions are those which contain 20 to 60 mol% of structural units of formula VI and/or VIa, 50 to 40 mol% of structural units of formula -$CH_2$-$CH_2$- and 0 to 50 mol% of structural units of formula -$CH_2$-CH(OH)-, wherein the sum of the mol% is 100 percent.

A further preferred composition to be used in the present invention is one wherein the thermoplastic polymer is a sulfated and/or sulfonatalkylated polymer of a partially hydroxylated polybutadiene, chloroprene or polyisoprene.

A preferred composition is one which contains 5 to 100 mol% of structural units of formula VIII and/or VIIIa

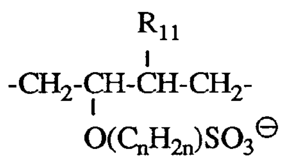

(VIII)

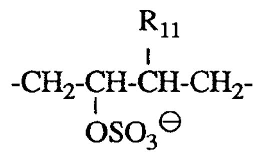

(VIIIa),

and 95 to 0 mol% of structural units of formula IX and/or IXa

$-CH_2-CH=CH-CH_2-$ (IX)

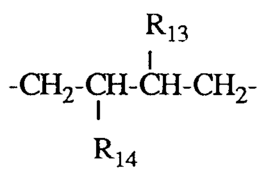

(IXa),

wherein $R_{13}$ and $R_{14}$ are each independently of the other H, OH or Cl, and $R_{11}$ and the group $-(C_nH_2)_n-$ has the meaning given hereinbefore including the preferred ones.

The above composition contains preferably 10 to 100 mol%, especially 20 to 60 mol%, of structural units of formula VIII and/or VIIIa and 90 to 0 mol%, especially 80 to 40 mol%, of structural units of formula IX and/or IXa. $R_{13}$ preferably represents H or Cl and $R_{14}$ preferably represents H.

A further preferrd composition to be used in the present invention is one wherein the thermoplastic polymer is a sulfated and/or sulfonatalkylated plyimide having structural elements of formula X

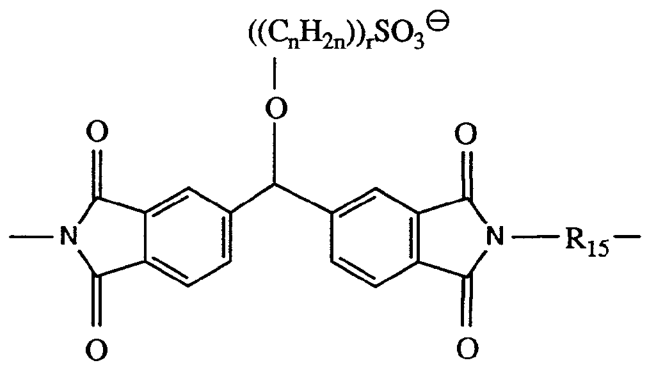

(X),

wherein $R_{15}$ stands for $C_6$-$C_{12}$-arylene or $C_5$-$C_8$-cycloalkylene, which are unsubstituted or substituted by $C_1$-$C_6$-alkyl or $C_1$-$C_6$-alkoxy, or the residue

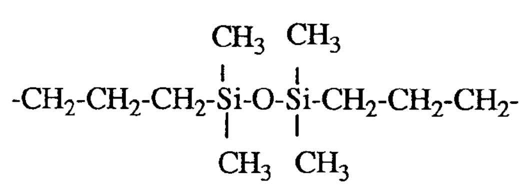

,

r means 0 or 1 and the group $-(C_nH_{2n})-$ has the meaning given hereinbefore including the preferred ones.

Another preferred composition to be used in the present invention is one in which the thermoplastic polymer represents a sulfated and/or sulfonatalkylated polyamide having structural units of formula XI

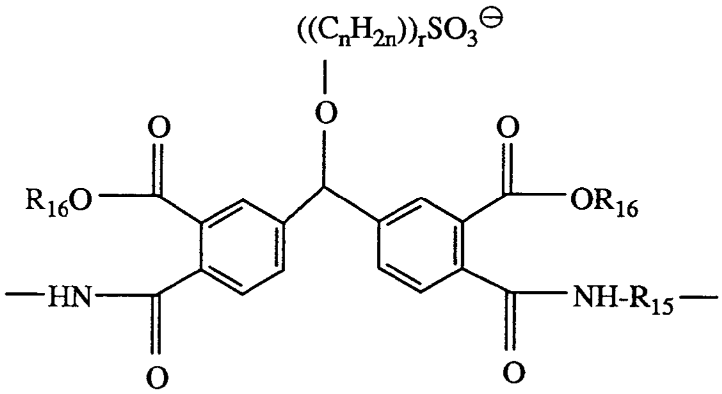

(XI),

wherein $R_{15}$ stands for $C_6$-$C_{12}$-arylene or $C_5$-$C_8$-cycloalkylene, which is unsubstituted or substituted by $C_1$-$C_6$-alkyl or $C_1$-$C_6$-alkoxy, or for the residue

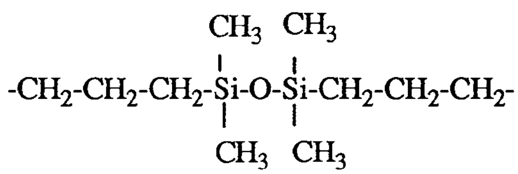

,

$R_{16}$ means $C_1$-$C_{12}$-alkyl, r stands for 0 or 1 and the group $-(C_nH_{2n})-$ has the meaning given hereinbefore including the preferred ones.

An especially preferred composition to be used in the present invention is one wherein the film forming thermoplastic polymer contains

a) 100 to 50 mol% of identical or different structural units of formula III and/or IIIa

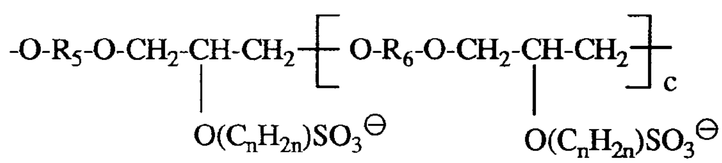

(III)

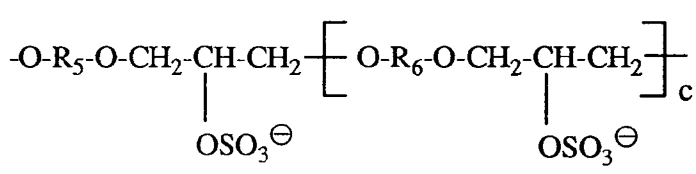

(IIIa)

and

b) 0 to 50 mol% of identical or different structural units of formula IV

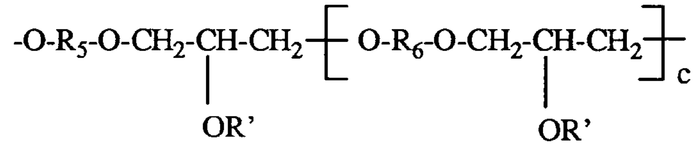

(IV)

based on the polyadduct, wherein $R_5$ and $R_6$ are each independently of the other a residue of a diol having aliphatic or aromatic diol groups, which residue is diminished by two hydroxyl groups, R' means H, $C_1$-$C_{20}$-alkyl, $C_1$-$C_{20}$-acyl or aminocarbonyl which is N-substituted by a $C_1$-$C_{20}$-hydrocarbon group, the group $-(C_nH_{2n})-$ has the meaning given hereinbefore including the preferred ones, and c is 0 or 1. Especially, the polymer contains 60 to 100 mol%, more preferably 60 to 90 mol%, of structural units of formula III and/or IIIa and 40 to 0 mol%, more preferably 40 to 10 mol%, of structural units of formula IV. R' is preferably hydrogen. This composition contains most preferably a polypyrrole in oxidized, polycationic form with structural units of formula I

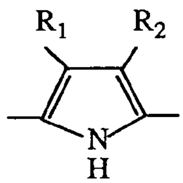

(I),

wherein $R_1$ and $R_2$ are each independently of the other hydrogen or $C_1$-$C_6$-alkyl, preferably hydrogen; or a polythiophene in oxidized, polycationic form with structural units of formula II

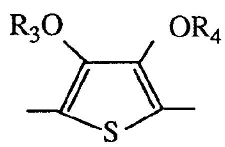

(II),

wherein $R_3$ and $R_4$ are each independently of the other linear or branched $C_1$-$C_6$-alkyl or $R_3$ and $R_4$ together represent methylene or the residue $-CHR_a-CHR_b-$, in which $R_a$ and $R_b$ are each independently of the other H, methyl or ethyl, preferably H.

A preferred composition to be used in the present invention is one wherein in formulae III, IIIa and IV $R_5$ and $R_5$ are each independently of the other a moiety of formula V

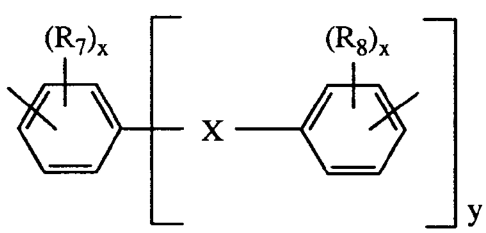

(V),

wherein X means a direct bond, $C_1$-$C_4$-alkylene, $C_1$-$C_{12}$-alkylidene, $C_5$-$C_8$-cycloalkylidene, -O-, S, SO-, $-SO_2-$, -CO-, $-CO_2-$, $-N(C_1$-$C_4$-alkyl)- or $-Si(CH_3)_2-$, $R_7$ and $R_8$ are each independently of the other H, halogene, $C_1$-$C_4$-alkyl or $C_1$-$C_4$-alkoxy, and x is 1 or 2 and y stands for 0 or 1.

Preferably, X represents a direct bond, methylene, ethylene, $C_2$-$C_6$-alkylidene, cyclohexylidene or cyclopentylidene, -O- or -S-. $R_7$ and $R_5$ stand preferably for H or methyl and y is preferably 1.

$R_5$ and $R_6$ are in particular the residue

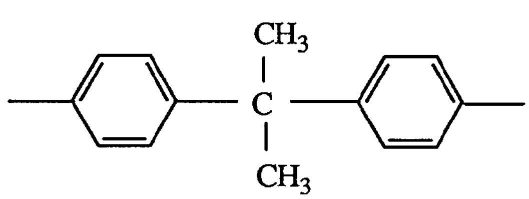

.

The structural units of the polypyrrole correspond most preferably to the residue of formula

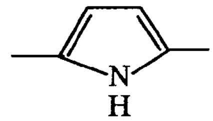

,

while those of the polythiophene correspond most preferably to the residue of formula

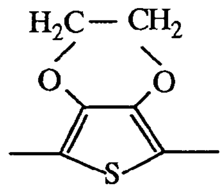

.

If the composition contains more than one polyanion of thermoplastic polymers having sulfated and/or sulfonatalkylated alcoholic groups, then two or three component mixtures are preferred.

The polyanion of component b) in the composition to be used in the present invention are derived from known polymer salts or from polymer salts which can be prepared by a known process. The polyanion relates to thermoplastic polymers containing sulfated alcoholic groups

$$-\overset{|}{\underset{|}{C}}-O-SO_3^{\ominus}\ M^{\oplus}$$

and/or sulfonatalkylated alcoholic groups

$$-\overset{|}{\underset{|}{C}}-O-(C_nH_{2n})-SO_3^{\ominus}M^{\oplus}$$

in the repeating structural units, wherein $M^{\oplus}$ stands for an alkalimetalic or ammonium cation.

The ammonium cation can be, for example, $NH_4^{\oplus}$, a protonated, primary, secondary or tertiary amine, or quarternary ammonium or pyridinium ion. The primary ammonium ion can contain 1 to 18, preferably 1 to 12, and most preferably 1 to 6, C-atoms, the secondary ammonium ion can contain 2 to 24, preferably 2 to 12, and most preferably 2 to 8, C-atoms, the tertiary ammonium ion can contain 3 to 30, preferably 3 to 18, and most preferably 3 to 12, C-atoms, and the quarternary ammonium ion can contain 4 to36, preferably 4 to 24, and most preferably 4 to 18, C-atoms.

Preferred polymer salts are those wherein $M^{\oplus}$ represents $Li^{\oplus}$, $Na^{\oplus}$ or $K^{\oplus}$, or $R_{17}R_{18}R_{19}R_{20}N^{\oplus}$, wherein $R_{17}$, $R_{18}$, $R_{19}$ and $R_{20}$ are each independently of the other H, unsubstituted or hydroxyl-substituted $C_1$-$C_{18}$-alkyl, phenyl, ($C_1$-$C_{12}$-alkyl)phenyl, ($C_1$-$C_{12}$-alkyl)benzyl, $C_5$-$C_7$-cycloalkyl, ($C_1$-$C_{12}$-alkyl)-$C_5$-$C_7$-cycloalkyl, or $R_{17}$ and $R_{18}$ are altogether tertamethylene, pentamethylene or 3-oxapentylene.

Preferred polymers in the present invention are those wherein at least one of $R_{17}$, $R_{18}$, $R_{19}$ and $R_{20}$ is not hydrogen. Particularly preferred are the polymers wherein $R_{17}$ to $R_{19}$ mean $C_1$-$C_6$-alkyl and $R_{20}$ is hydrogen.

$R_{17}$ to $R_{20}$ as alkyl can be linear or branched and contain preferably 1 to 12, more preferably 1 to 6, C-atoms. Examples of such alkyl are methyl, ethyl, n- and i-propyl, n-, i- and t-butyl, pentyl, hexyl, octyl, nonyl, decyl, undecyl, dodecyl, tetradecyl, hexadecyl and octadecyl.

$R_{17}$ to $R_{20}$ as hydroxyalkyl can be linear or branched and contain preferably 2 to 18, more preferably 2 to 12, and most preferably 2 to 6, C-atoms. Examples of such hydroxyalkyl are 2-hydroxyeth-1-yl, 1- or 2-hydroxyprop-3-yl, 1-hydroxybut-4-yl and 1-hydroxyhex-6-yl.

Examples of alkylphenyl and alkylbenzyl are methylphenyl, dimethylphenyl, ethylphenyl, n- or i-propylphenyl, n-, i- or t-butylphenyl, hexylphenyl, octylphenyl, decylphenyl, dodecylphenyl and the corresponding alkylated benzyl residues.

$R_{17}$ to $R_{20}$ as cycloalkyl stands preferably for cyclopentyl or cyclohexyl.

$R_{17}$ to $R_{20}$ as alkylcycloalkyl are preferably ($C_1$-$C_{12}$-alkyl)cyclopentyl or -cyclohexyl such as for example methyl- or ethylcyclopentyl or -cyclohexyl.

In particular, $R_{17}$ to $R_{20}$ stand for $C_1$-$C_6$-alkyl, or $R_{17}$ to $R_{19}$ are $C_1$-$C_6$-alkyl and $R_{20}$ is hydrogen.

The polymer salts eligible for use in the preparation of the composition to be used in the present invention are known materials, or can be prepared according to generally known methods. Depending on the kinds of polymers and the process conditions to be used, the hydroxyl groups of the monomers to be used can be protected using conventional protecting groups. Polymers containing hxdroxyl groups are well known in the art, or can be obtained according to known processes.

The thermoplastic polymer salts eligible for use in the preparation of the composition to be used in the present invention can be obtained in known manner wherein a thermoplastic polymer which contains alcoholic groups

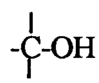

in repreating structural unit is reacted in an inert solvent with a sulfating reagent (for example, pyridine.$SO_3$) or a sulton of formula

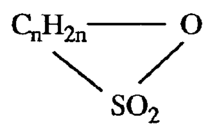

in the presence of an alkali metal base or an amine and thereafter the obtained polymer salt is isolated. The reaction can be carried out stepwise; firstly with a sulfating reagent or a sultone in an amount less than equimolar quantity, then with the sulton or sulfating reagent to obtain a polymer containing sulfate groups and/or aulfonatalkylating groups.

Suitable inert solvents are preferably polar aprotic solvents, the choice of solvent depending on the solubility of the hydroxyl group containing polymer. The solvents may be used alone of as a mixture of at least two solvents. Illustrative of suitable solvents are: ethers such as dibutyl ether, tetrahydrofuran, dioxan, methylene glycol, dimethylethyleneglycol, dimethyl diethylene glycol, diethyl diethylene glycol, dimethyl triethylene glycol, halogenated hydrocarbons such as methylene chloride, chloroform, 1,2-dichloroethane, 1,1,1-trichloroethane, 1,1,2,2-tetrachloroethane, and lactones such as $\gamma$-butyrolactone, o-valerolactone and pivalolactone, carboxamides and lactams such as N,N-dimethylformamide, N,N-diethylformamide, N,N-dimethylacetoamide, N-methyl-$\gamma$-butyrolactam, N-methyl-$\epsilon$-caprolactam, N-methylpyrrolidone, N-acetylpyrrolidone, tetramethylurea, hexamethylphosphoramide, sulfoxides such as dimethyl sulfoxide, sulfones such as dimethyl sulfone, diethyl sulfone, trimethylen sulfone, tetramethylene sulfone, N-methylpyrrolidine, N-methylpiperidine, N-methylmorpholine, substituted benzenes such as benzonitrile, chlorobenzene, o-dichlorobenzene, 1,2,4-trichlorobenzene and nitrobenzene.

The reaction temperature is, for example, 40° to 200°C, preferably 60° to 150°C. After finishing the reaction, the polymer salt is precipitated, filtered and washed in a non-solvent liquid, in water advantageously, and dried.

Sulfonatalkylated phenoxy resins are described, for example, in BE-B 847 353. Sulfonatalkylated polymers are described, for example, in EP-A-0 566 536 and FR-B 1 584 814. Sulfated polymers are described, for example, in US-A 5 061 401.

The eligible salts of polymers containing sulfated and/or sulfonatalkylated hydroxyl groups have thermoplastic properties. Compared with the starting polymers, their glass transition temperature is substantially unchanged and they are distinguished by their mechanical properties, for example, by high tensile and flexural strength and high flexibility. They are highly suitable polyanions for the preparation of electroconductive polymer films to be used for the production of the protein adsorbed shapable electroconductive polymer films of the present invention.

The compositions to be used in the present invention are prepared in a manner known per se by electrochemically polymerizing a heteroaromatic compound or aniline in an organic, aqueous-organic or aqueous solution in the presence of at least one salt of a film-forming thermoplastic polymer containing sulfated alcoholic groups

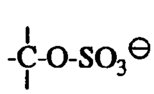

and/or sulfonatalkylated alcoholic groups

$$-\overset{|}{\underset{|}{C}}-O-(C_nH_{2n})-SO_3^{\ominus}$$

in repeating structural units, wherein the group $-(C_nH_{2n})-$ stands for linear or branched $C_2$-$C_{12}$-alkylene having 2 to 5 C-atoms in the carbon chain, which alkylene is unsubstituted or substituted by $C_1$-$C_4$-alkoxy, and subsequently removing the composition from the anode.

The electrolysis can be carried out under potentiostatic or galvanostatic conditions. As anode materials, inert metals such as Ti, Ni, Pt and steel, or ITO (indium tin oxide) glass, or not inert metals such as Al (see, for example, DE-A 906 563) can be used The current density can be, for example, 0.005 to 50 mA/cm$^2$, preferably 0.05 to 20 mA/cm$^2$, and most preferably 0.05 to 10 mA/cm$^2$.

The concentration of the polymer salts can be 0.05 to 1 mol/l, preferably 0.05 to 0.5 mol/l, based on the reaction mixture. The concentration of the polyheteroaromatic compound or aniline can be 0.02 to 10 vol%, preferably 0.1 to 5 vol%, based on the volume of the reaction mixture.

Suitable organic solvents are those mentioned hereinbefore. Preferred solvents are alcohols such as for example alkanols having 1 to 12 carbon atoms which may be substituted by $C_1$-$C_4$-alkoxy. Examples of such alcohols are methanol, ethanol, n- and i-propanol, n-, i- and t-butanol, pentanol, hexanol, heptanol, octanol, decanol, dodecanol, methoxyethanol, ethoxyethanol, diethyleneglycolmonomethyl- or-monoethylseter, 1-methoxypropane-2- or -3-ol. Other preferred solvents are carbonic acid esters such as propylene carbonate.

If reactants are sufficiently soluble, the electrochemical polymerization can be carried out in an aqueous or aqueous-organic solution. It is advantageous to use a buffer in the solution. Suitable buffers are, for example, alkylammonium phosphate having 1 to 3, preferably 2 or 3, alkyl residues in the ammonium group which can contain 1 to 6, preferably 1 to 4, carbon atoms. Examples of such alkylammonium phosphates are trimethyl-, triethyl-, tri-n-propyl- and tri-n-butylammonium phosphate. Cation exchangers in protonated form are also suitable buffers.

After finishing electrolysis, the composition in film form to be used in the present invention is removed from the anode and washed with solvents. The film has a thickness of 0.1 μm to 0.3 cm, preferably 0.5 μm to 0.1 cm, more preferably 0.5 μm to 500 μm, and most preferably 1 μm to 200 μm. The thickness depends essentially on the duration of electrolysis.

The compositions to be used in the present invention have low glass transition temperatures, so that even where polyanion concentration is low, processing by thermoplastic moulding or other methods for thermoplastic polymers is possible without loss of electrical conductivity. The films to be used in the present invention are therefore shapable.

Upon necessity, the electrical conductivity of the above films can be increased by drawing at a temperature below the melting or decomposing temperature, preferably in the range of the glass transition temperature, especially in the range of 20°C below or above the glass transition temperature.

The maximum size of the above films can be controlled by properly chosing the size of the electrode to be used for the electropolymerization of the films. Herein, films of a certain width and an arbitrary length can be prepared by employing a rotating drum type electrode.

The protein to be used in the present invention may have molecular distinguishing ability.

Herein, the molecules to be distinguished may be small molecules such as mono- or disaccharides as well as large ones such as polymeric compounds, or even microorganisms such as antigens and antibodies. Typical examples of the proteins to be used in the present invention are proteins for biosensor applications, proteins for bioreactor applications, and proteins for immunosensor applications.

Suitable proteins for biosensor applications are, for example, flavoenzymes such as glucose oxidase, D-amino acid oxidase, L-amino acid oxidase, choline oxidase, xanthine oxidase, sarcosine oxidase, L-lactate oxidase, pyruvate oxidase, ascorbate oxidase, cholesterol oxidase and the like, quinoproteins such as fructose dehydrogenase, sorbitol dehydrogenase and the like, and NAD enzymes such as alcohol dehydrogenase, lactate dehydrogenase, maltose dehydrogenase, glutamate dehydrogenase, glycerol dehydrogenase, 11B hydroxysteroid dehydrogenase, nitrate reductase, oestradiol 17B dehydrogenase, aminoacids dehydrogenase and others. Any protein to be used for biosensors can be used for the protein adsorbed shapable electroconductive polymer film of the present invention.

Suitable proteins for bioreactor applications are, for example, enzymes for peptide synthesis such as α-chymotripsin, tripsin, papain, pepsin, subtilisin, thermoase, thermolysin and the like, lipase for glyceride synthesis and others. Any protein to be used for bioreactor applications can be used for the protein adsorbed shapable electroconductive polymer film of the invention.

Suitable proteins for immunosensor applications are anitigens and antibodies, such as for example human serum albumin (HSA), immunoglobin G (IgG), IgA, IgM, IgD, IgE, protein A, theophyline, human chorionic gonadotropin (HCG), temor antigen a-fetoprotein, cardiolipin and others. Any foreign protein in the body can be determined by immunoassay, so that there is no limitation in the proteins to be used for immunosensor applications.

A further object of the invention is a process for preparing a protein adsorbed shapable electroconductive polymer film, wherein a shapable electroconductive polymer film as described above is contacted with an aqueous protein solution.

The protein adsorbed shapable electroconductive polymer film according to the present invention can be fabricated in the following procedure.

Firstly, a shapable electroconductive polymer film as described above is cut to a size in accordance with requirements, followed preferably by cleaning using a solvent in order to eliminate organic contamination which may possibly block the adsorption sites of proteins. The cleaning can be carried out by keeping the electroconductive film immersed in a solvent for 1 to 100 hours, preferably 3 to 24 hours, at room temperature with continuous stirring condition. As solvents therefor, organic solvents including those mentioned hereinbefore are preferred. Particularly preferred are ketones such as acetone, methylethyl ketone, methylisobutyl ketone, cyclohexanone and others, benzene and its derivatives such as haloganated benzenes, e.g., fluoro- and chlorobenzene, nitrobenzene, alkylated benzenes, e.g., toluene, xylene, ethylbenzene and others, alcohols such as methanol, ethanol, n- and i-propanol, n-, i- and t-butanol, benzyl alcohol and others, and nitriles such as acetonitrile, propionitrile, benzonitrile and others. The solvents can be used alone or as a mixture. After the cleaning, the electroconductive polymer film is washed thoroughly with a fresh solvent, the solvent being one or a mixture of the above organic solvents, then washed further with an alcohol and deionized water. Thereafter, the film is dried under vacuum for 1 to 10 hours, preferably 2 to 3 hours. It is preferred to store the thus cleaned electroconductive polymer film in a closed container at room temperature so as to avoid contamination.

In order to increase the amount of the protein adsorbed on the shapable electroconductive polymer film, it is effective to treat it by plasma etching technique. As equipment therefor, there is no limitation as far as it enables plasma etching. Any gas used conventionally for plasma treatment, for example, $N_2$ gas, air, $O_2$ gas or the like can be used for this purpose. The duration of the plasma etching can be chosen according to requirements. It can be varied, for example, from 1 minute to 10 hours or more.

Then, the above prepared, cleaned, or cleaned and plasma etched shapable electroconductive polymer film is subjected to protein adsorption. Said film is incubated in a phosphate buffer solution containing a desired protein for 1 to 10 hours, preferably 3 to 24 hours, preferably at room temperature. The pH of the above buffer solution is chosen such that the protein is stable. For example, pH 7.0 is preferred for glucose oxidase, while pH 6.0 is preferred for fructose dehydrogenase. The concentration of the protein is preferably 0.01 to 100 mg/ml, more preferably 0.1 to 50 mg/ml, and most preferably 1 to 10 mg/ml. After the completion of incubation, the protein adsorbed electroconductive polymer film is preferably incubated for another 2 to 3 hours in another buffer solution of a stable pH, for example, 6.0 for glucose oxidase and 4.5 for fructose dehydrogenase, in order to desorb loosely attached protein, whereby a protein adsorbed shapable electroconductive polymer film according to the invention is fabricated.

It is effective to apply a potential to the electroconductive polymer film during the above protein adsorption in order to increase the amount of the adsobed protein and accelerate the rate of adsorption. Using this technique the time required for completing the adsorption reaction is shortened to such a short time as several minutes. This potential-aided adsorption can be accomplished as follows: The above prepared, cleaned, or cleaned and plasma etched electroconductive polymer film is made an electrode by attaching a metal strip, such as a thin aluminum sheet or the like, using a carbon paste or the like. A conventional system of three electrodes composed of a Pt wire counter electrode, a Ag/AgCl reference electrode and the above prepared electroconductive polymer film working electrode is set up in a phosphate buffer solution containing a protein. The pH and protein concentration are chosen in the same manner as described above. Then, a potential is applied between the working electrode and the reference electrode for 1 to 30 minutes, preferably 5 to 10 minutes. The potential is preferably from -1 to + 1 V, more preferably from -0.8 to + 0.8 V, measured against the Ag/AgCl reference electrode. The optimum potential is dependent on the protein, so that it is determined experimentally by observing the rate of adsorption and the activity of the adsorbed protein. After the completion of the above potential-aided adsorption, the protein adsorbed shapable electroconductive polymer film is washed thoroughly with water and a buffer of the same pH. Subsequently, the protein adsorbed section of the above film is cut and preferably incubated for another 2 to 3 hours in another buffer solution of a stable pH in order to desorb loosely attached protein, thereby yielding a protein adsorbed shapable electroconductive polymer film of the present invention. There may be adsorbed for example 0.1 to 50 µg, more preferably 0.2 to 10 µg protein.

As described above, according to the present invention, there is provided a protein adsorbed shapable electroconductive polymer film which production process only requires the step of incubating an electroconductive polymer film in an aqueous solution containing a protein to be adsorbed thereon. Since no electrolysis is needed for the immobilization of proteins, the production process is simplified, so that the production cost can be reduced greatly and undesirable effects caused by electrolysis, such as denaturation of proteins, can be avoided. Moreover, if desired, the amount and rate of the protein adsorption can be drastically increased by providing a counter electrode in the solution and applying a potential between the shapable electroconductive polymer film and the counter electrode, said potential lying far below that causing denaturation or side reactions of the protein. Furthermore, since the protein adsorbed shapable electroconductive film of the present invention is flexible and shapable, it can be easily applied in various fields such as biosensors, bioreactors, immunosensors and the like.

Therefore, the protein adsorbed shapable electroconductive polymer film of the present invention is far more advantageous over the prior art biosensor materials, bioreactor materials, immunosensor materials and others.

The following examples explain the invention in more detail wthout limiting the scope.

Example 1

(1) Preparation of shapable electroconductive polymer film:

28.4 g of polyadduct of bisphenol A diglycidyl ether and bisphenol A having an average molecular weight of about 13000 (degree of polymerization ca. 50) are dissolved at 50°C in 100 ml of dimethyl formamide (DMF). To this solution are added 5.3 g of sulfur trioxide/pyridine complex in 20 ml of DMF. After 5 hours, the reaction mixture is cooled to 5°C and $(n\text{-}C_4H_9)_3N$ is added, the mole number of said amine being the same as that of the above sulfur trioxide/pyridine complex. The reaction mixture is poured into water and the precipitated polymer salt is isolated by filtration, washed with water and dried, thereby yielding 28.6 g (73 % in yield) of polymer salt with Tg of 81°C in which sulfation takes place every third OH group. Then, 12 g of the thus prepared partially sulfated polymer, 10 ml of pyrrole and 2 ml of water are dissolved in 200 ml of propylene carbonate. The solution is transferred to an electrolysis cell fitted with a rotating stainless steel anode and a fied stainless steel cathode. After 50 minutes, a 100 μm thick film is deposited at a current density of 2 $mA/cm^2$. After extraction with propylene carbonate and ethanol and vacuum drying, the yield is 282 mg. The cinductivity is 20.0 S/cm. The glass transition temperature is 63°C. The electroconductive polymer film prepared above will be referred to as SEC film (Shapable Electro Conductive film).

(2) Cleaning of SEC film

The above prepared SEC film is cleaned firstly with ethanol for 2 hours, then with acetonitrile for 1 hour, with continuous stirring condition. After thorough washing with fresh solvents (ethanol and acetonitrile) and deionized water, SEC film is vacuum dried for 3 hours and stored at room temperature in a closed bottle.

(3) Physical adsorption of enzyme

The above cleaned SEC film is cut into 1 cm x 0.5 cm pieces, and the pieces are respectively incubated in a phosphate buffer solution of pH 7.0 containing 5 mg/ml of glucose oxidase (GOD, 168 U/mg, 80 % protein, a commercial product of Sigma) from Aspergillus niger (type III) for 0.01 to 70 hours. After thorough washing with water and a buffer of the same pH, the enzyme adsorbed films are respectively incubated for another 2 to 3 hours in a buffer solution of pH 6.0 in order to desorb loosely attached enzyme.

(4) Measurement of activity of adsorbed enzyme

The activity of the above GOD adsorbed SEC film pieces is measured by the method described in Toyobo Enzymes Manual (published by Toyobo, Osaka, 1991).

(5) Measurement of enzyme adsorbed on SEC film

A highly sensitive protein assay method utilizing ophthaldialdehyde is used in order to measure the amount of the protein adsorbed on the film. Every sample and blank (only SEC film pieces) are at least triplicated. Highly deviated (more than 25 % from the mean value) data are not considered for calculation, for those cases, the whole experiment is repeated. All data are the means of 3 to 5 samples.

(6) QCM (Quartz Crystal Microgravimetry) measurement of adsorbed enzyme

A very thin (1 to 2 μm) film of polypyrrole is prepared on the Pt surface of a quartz crystal under the same condition as thick SEC film and processed in the same way as thick SEC film. 3 ml of a phosphate buffer solution of pH 7.0 is put into the specially made cell provided on the polypyrrole/crystal and waited for a few minutes until the frequency is stabilized Then, 1 ml of the same buffer is further added into the above cell in order to check the stability of the frequency. Subsequently, 1 ml of a GOD solution is added

into the cell to a final concentration of 5 mg/ml and the frequency decrease with time is recorded. After a desired time, the polypyrrole/crystal is washed with the above buffer carefully by gradually replacing the GOD containing buffer with the fresh one. Within 4 to 5 minutes the frequency becomes stable with a little increased frequency (equivalent to 20 to 40 % protein adsorbed) due to th desorption of loosely adsorbed protein. The amount of the adsorbed protein is calculated from the change of frequency from the initial to the final values.

(7) Results

In table 1 is shown the time dependence of the protein adsorption. Within 3 hours more than 70 % of the total adsorption is completed. By the QCM study it is observed that nearly 50 % of the protein adsorption is completed within 3 to 4 minutes (Table 4).

Table 1:

| Time Dependence of Physical Adsorption | | |
|---|---|---|
| Time (hr) | Protein (ug/$cm^2$) | Activity (mU/$cm^2$) |
| 2.4 | 0.93 | 1.63 |
| 24 | 1.29 | 2.17 |
| 48 | 1.57 | 2.54 |
| 72 | 1.71 | 2.96 |

Example 2:

(1) and (2): Preparation and cleaning of SEC film is carried out in the same manner as in Example 1 (1) and (2).

(3) Physical adsorption of enzyme

Cleaned SEC film is cut into 1 cm x 0.5 cm pieces, and they are respectively incubated in a phosphate buffer solution of pH 6.0 containing fructose dehydrogenase (FDH, 23 U/mg, 70 % protein, a commercial product of Toyobo) from Gluconbacter sp. for 22 hours. After thorough washing with water and a buffer of the same pH, the enzyme adsorbed SEC film pieces are respectively incubated for another 2 to 3 hours in a buffer solution of pH 4.5 in order to desorb loosely attached enzyme.

(4) Measurement of activity of adsorbed enzyme

The activity of the adsorbed FDH is measured according to the method prescribed in Toyobo Enzymes Manual (published by Toyobo, Osaka, 1991).

(5) Measurement of enzyme adsorbed on SEC film

The amount of the adsorbed FDH is assayed in the same manner as in Example 1(5) except that the FDH determination is done by comparing with BSA.

(6) Stability of enzyme on SEC film surface

The stability of the FDH adsorbed on SEC film surface is examined in dry form and in an aqueous solution by comparing with that of free FDH under the same condition.

(7) Results

(i) Amount and activity of adsorbed enzyme:

In the above physical adsorption as described in (3), FDH is adsorbed on the SEC film in an amount of 4.8 $\mu g/cm^2$ and activity of 130 $mU/cm^2$.

(ii) Stability of adsorbed enzyme:

In Table 2 is shown the time stability of the FDH adsorbed on SEC film. For comparison, the stability of free enzyme is also shown. Results indicate that the adsorbed FDH is more stable than free FDH and loss of activity is mainly due to the inactivation of the enzyme in the aqueous solution. The adsorbed FDH is stable even after 20 days in the aqueous solution, retaining more than 80 % of the initial activity. The adsorbed FDH is perfectly stable when stored dry in a freezer. Therefore, enzymes adsorb strongly on SEC film and do not desorb even after a long time incubation in aqueous solution.

Table 2 Stability of FDH/SEC in Aqueous Solutions

| Time (day) | Relative Activity (%) | | |
|---|---|---|---|
| | Free FDH | FDH/SEC | FDH/SEC (dry form) |
| 1 | 100 | 100 | 100 |
| 4 | 67 | - | - |
| 5 | - | 89 | 100 |
| 6 | 51 | - | - |
| 8 | 48 | - | 100 |
| 15 | 39 | 85 | 100 |
| 19 | 38 | 85 | 100 |

(Both free (0.1 mg/ml) and adsorbed FDH (FDH/SEC) were incubated in a pH 4.5 buffer solution at 4°C.)

Example 3:

(1), (2): Preparation and cleaning of SEC film is carried out in the same manner as in Example 1(1) and (2).

(3) Potential aided adsorption of enzyme

A conventional system of tree electrodes composed of a Pt wire counter electrode, a Ag/AgCl reference electrode and a SEC film working electrode is set up in a phosphate buffer of pH 7.0 containing 5 mg/ml of GOD. Potential aided adsorption is carried out on a SEC film piece by applying a potential between the working and reference electrodes for 5 to 10 minutes. After thorough washing with water and the buffer solution, the enzyme adsorbed section of the electrode is cut and incubated in a buffer solution of pH 6.0 for another 2 to 3 hours in order to desorb loosely attached enzyme.

(4) Measurement of activity of adsorbed enzyme

The activity of the GOD adsorbed on SEC film pieces is measured in the same manner as in Example 1(4). However, because the absorbance is sometimes found to increase continuously with time due to the desorption of the enzyme in the assay solution for specimens prepared by potential-aided adsorption, the activity of free enzyme in the solution is calculated from the slope of time vs. absorbance curve and deduced from the initial activity. This way, only the adsorbed enzyme activity is measured.

(5) Measurement of enzyme adsorbed on SEC film

The assay of the GOD adsorbed on SEC film pieces is done by employing BCA* (Bicinchoninic Acid) protein method. Each of the enzyme adsorbed SEC film pieces is incubated overnight in CHAPS (3-((3-cholamidopropyl)-dimethylammonio)-1-propane sulfonate, from Dojin Chemical Co.) solution (0.25 % to 1 %) followed by sonication for two minutes. Sometimes, a protein adsorbed film piece is incubated in a 47.5 % ethanol solution in order to desorb the protein completely. Then, the desorbed protein is measured by BCA* reagent (a commercial product of PIERCE) according to the method prescribed by PIRCE. For the determination of the adsorbed GOD, commercially supplied GOD (considering 80 % protein) is used for calibration. Every sample and blank (only SEC film pieces) are at least triplicated. All the data are the means of 3 to 5 samples.

(6) QCM measurement of adsorbed enzyme

QCM measurement is carried out in the same manner as in Example 1(6) except that a three electrode system is set up on the top of the crystal, where polypyrrole coated crystal acts as a working electrode, and then a potential is applied.

(7) Results

(i) Amount and activity of adsorbed enzyme

In Table 3 is shown the potential dependent adsorption of GOD. Both the adsorbed GOD and the activity thereof are highly potential dependent. In a solution of pH 7.0, GOD is negatively charged, so that application of a positive potential assists very fast protein adsorption. In the lower potential range of from -0.2 to 0.2 V, the protein adsorption is the lowest. By increasing the potential in the positive direction, the protein adsorption increases sharply and continues to increase even beyond 0.8 V. Activity curve saturates after 0.6 V mainly due to the inactivation of the inner enzyme layer. However, in the negative direction, the protein adsorption as well as the activity of the adsorbed protein increases from - 0.4 V. It is preferred to apply a potential of from 0.4 V to 0.6 V for the protein adsorption.

(ii) QCM measurement of adsorbed enzyme

Table 3 shows the time dependent adsorption of GOD on a very thin (1 μm) polypyrrole film prepared on the crystal. The amount of adsorption is calculated from the frequency drop. In the case of potential-aided adsorption nearly 80 % of the adsorption completes within 2 minutes and becomes almost saturated within 5 minutes, while in the case of physical adsorption nearly 50 % of the adsorption completes within 3 to 4 minutes. The adsorption of GOD at 0.4 V is about 3.4 μg/$cm^2$.

Table 3

| Potential Dependence of Protein Adsorption | | |
|---|---|---|
| Potential (V) | Protein (ug/$cm^2$) | Activity (mU/$cm^2$) |
| -0.4 | 4.5 | 2.3 |
| -0.2 | 1.6 | 1.5 |
| 00 | 1.4 | 1.4 |
| +0.2 | 2.6 | 1.8 |
| +0.4 | 3.4 | 2.4 |
| +0.6 | 4.8 | 2.7 |
| +0.8 | 5.6 | 2.9 |

(Potential is applied in a pH 7.0 phosphate buffer containing 5 mg/ml of GOD for 10 minutes.)

Table 4

| QCM Results of Protein Adsorption on Very Thin PPy Membrane (1 μm) Prepared on Crystal: | | | |
|---|---|---|---|
| Time (min) | Adsorbed GOD (ug/$cm^2$) | | |
| | 0.4 V | 0.2 V | 0 V |
| 1 | 2.1 | 1.2 | 0.7 |
| 2 | 2.8 | 1.8 | 1.0 |
| 3 | 3.0 | 2.0 | 1.1 |
| 5 | 3.2 | 2.1 | 1.2 |
| 10 | 3.2 | 2.2 | 1.3 |
| 180 | - | - | 1.7 |

## Example 4

(1),(2): Preparation and cleaning of SEC film is carried out in the same manner as in Example 1(1) and (2).

(2') Plasma etching of SEC film

The above prepared SEC film is cut into 8 pieces. Two of them are used as control, and the others are treated by $N_2$ plasma (2 pieces), air plasma (2 pieces) and $O_2$ plasma (2 pieces) respectively for 30 minutes.

(3) Physical adsorption of GOD or FDH

The above prepared plasma etched SEC film pieces are subjected to the adsorption of GOD or FDH according to the procedure described in Example 1(3) or Example 2(3).

(4)Measurement of activity of adsorbed GOD or FDH is carried out in the same manner as in Example 1(4) or Example 2(4).

(5) Results

Table 5 shows the enzyme adsorption on the plasma etched SEC film. In the case of air plasma (30 min.) the activity of the adsorbed GOD increases about 1.6 times, whereas that of the adsorbed FDH increases about 4.3 times. On the other hand, for $N_2$ plasma, the activity of GOD and FDH both increases about 2 times. Oxygen plasma also increases the activity of the adsorbed GOD and FDH as shown in this table.

Table 5

| Adsorption of Enzyme on Plasma Etched Film | | | |
|---|---|---|---|
| Film | Adsorption time (hr) | Adsorbed GDO Activity (mU/cm$^2$) | Adsorbed FDH Activity (mU/cm$^2$) |
| Normal Sec film | 22 | 1.3 | 130 |
| $N_2$ plasma (30 min) | 22 | 2.4 | 230 |
| Air plasma (30 min) | 22 | 2.2 | 550 |
| $O_2$ plasma (30 min) | 22 | 1.9 | 380 |

Example 5

(1) Preparation of SEC film

2 g (3,6 mmol) of a tri-n-butylammonium salt of a poly(β-hydroxyether) which is partially sulfated by 30 mol% and has a molecular weight 28000, prepared by the polyaddition of bisphenol-A-diglycidylether and bisphenol-A, and 0.2 ml (0.75 vol%) of 3,4-ethylenedioxy-thiophene (EDT) are dissolved in 30 ml of propylene carbonate at room temperature and introduced into an electrolysis cell which is filled with an inert gas. This cell is provided with electrodes made of stainless steel. In order to ensure homogeneous mixing of the electrolyte solution, a rotating anode is used. The cathode is made net-form in order to provide an outlet for gaseous by-products. A Ag/AgCl reference electrode is connected to the anode in order to control the oxidation potential of the electropolymerization. The electrolysis is carried out at a current density of 0.1 mA/cm$^2$ at a potential of 1.5 to 1.6 V against the reference electrode for 2.6 hours. The deposited film is mechanically removed from the anode, extracted in acetonitrile for 1 hour under reflux condition and dried at 50°C in a high vacuum. The thus obtained film has an electroconductivity of 70 S/cm.

(2) Cleaning of SEC film

The above SEC film is cleaned in the same manner as in Example 1(2).

(3) Physical adsorption of enzymes

GOD and FDH are adsorbed on SEC film pieces in the same manner as in Example 1(3) and Example 2(3).

(4) Measurement of activity of adsorbed enzymes

The activity of the adsorbed GOD and FDH is measured in the same manner as in Example 1(4) and Example 2(4).

(5) Results

In Table 6 are shown the results of the enzyme adsorption on 3,4-ethylenedioxy-thiophene based SEC film (PEDT). For comparison, the results obtained from polypyrrole based SEC film (PPy) are also shown. The adsorption of GOD on PEDT film is 2.5 times higher than that of PPy based film, on the other hand, the adsorption FDH on PEDT based film is lower than that of PPy based film.

Table 6

| Adsorption of Enzymes on PEDT based SEC film | | | |
|---|---|---|---|
| Film | Adsorption time (hr) | Adsorbed GOD Activity ($mU/cm^2$) | Adsorbed FDH Activity ($mU/cm^2$) |
| PPy based SEC film | 22 | 1.3 | 130 |
| PEDT based SEC film | 22 | 3.3 | 62 |

## Claims

1. A protein adsorbed shapable electroconductive polymer film comprising
   1) a film made of a composition comprising
      a) at least one polyheteroaromatic compound or aniline in oxidized, polycationic form, and
      b) at least one polyanion of a film-forming thermoplastic polymer containing sulfated alcoholic groups

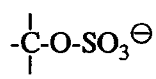

      and/or sulfonatalkylated alcoholic groups

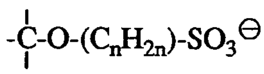

      in repeating structural units, wherein the group $-(C_nH_{2n})-$ is linear or branched $C_2$-$C_{12}$-alkylene containing 2 to 5 C-atoms in the carbon main chain, the alkylene being unsubstituted or substituted by $C_1$-$C_4$-alkoxy; and
   2) a protein adsorbed on the surface of said film.

2. A protein adsorbed shapable electroconductive polymer film according to claim 1, wherein the polyheteroaromatic compound is formed from 5-membered rings which contain 1 to 3 hetero atoms selected from the group consisting of O, S and N, and the carbon atoms of which are unsubstituted or substituted by $C_1$-$C_{18}$-alkyl.

3. A protein adsorbed shapable electroconductive polymer film according to claim 2, wherein the 5-membered ring is selected from the consisting of pyrrole, thiophene, furan, 2,2'-bipyrrole, 2,2'-bithiophene, 2,2'-bifuran, thiazole, oxazole, thiadiazole and imidazole.

4. A protein adsorbed shapable electroconductive polymer film according to claim 2, wherein the polyheteroaromatic compound has the repeating structural units of formula I

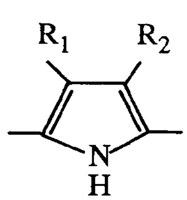

(I),

   wherein $R_1$ and $R_2$ are each independently of the other hydrogen or $C_1$-$C_{16}$-alkyl.

5. A protein adsorbed shapable electroconductive polymer film according to claim 2, wherein the polyheteroaromatic compound has the repeating structural units of formula II

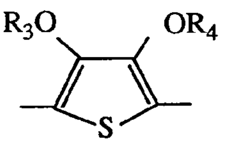

(II),

wherein $R_3$ and $R_4$ are each independently of the other linear or branched $C_1$-$C_{18}$-alkyl or $C_2$-$C_{18}$-alkoxyalkyl; or $C_3$-$C_8$-cycloalkyl, phenyl or benzyl, which may be unsubstituted or substituted by $C_1$-$C_6$-alkyl, $C_1$-$C_6$-alkoxy or halogen; or $R_3$ and $R_4$ together form linear $C_1$-$C_6$-alkylene which may be unsubstituted or substituted by $C_1$-$C_6$-alkyl, $C_1$-$C_6$-alkoxy, halogen, $C_3$-$C_8$-cycloalkyl, phenyl, benzyl, $C_1$-$C_4$-alkylphenyl, $C_1$-$C_4$-alkoxyphenyl, halogenphenyl, $C_1$-$C_4$-alkylbenzyl, $C_1$-$C_4$-alkoxybenzyl or halogenbenzyl.

**6.** A protein adsorbed shapable electroconductive polymer film according to claim 5, wherein $R_3$ and $R_4$ as alkyl are $C_1$-$C_{12}$-alkyl.

**7.** A protein adsorbed shapable electroconductive polymer film according to claim 6, wherein $R_3$ and $R_4$ are $C_1$-$C_8$-alkyl.

**8.** A protein adsorbed shapable electroconductive polymer film according to claim 6, wherein $R_3$ and $R_4$ are $C_1$-$C_4$-alkyl.

**9.** A protein adsorbed shapable electroconductive polymer film according to claim 5, wherein $R_3$ and $R_4$ as alkoxyalkyl are $C_1$-$C_{17}$-alkoxymethyl or $C_1$-$C_{16}$-alkoxyethyl.

**10.** A protein adsorbed shapable electroconductive polymer film according to claim 9, wherein $R_3$ and $R_4$ are $C_1$-$C_{12}$-alkoxymethyl or $C_1$-$C_{12}$-alkoxyethyl.

**11.** A protein adsorbed shapable electroconductive polymer film according to claim 9, wherein $R_3$ and $R_4$ are $C_1$-$C_8$-alkoxymethyl or $C_1$-$C_7$-alkoxyethyl.

**12.** A protein adsorbed shapable electroconductive polymer film according to claim 9, wherein $R_3$ and $R_4$ are $C_1$-$C_4$-alkoxymethyl or $C_1$-$C_4$-alkoxyethyl.

**13.** A protein adsorbed shapable electroconductive polymer film according to claim 5, wherein $R_3$ and $R_4$ as cycloalkyl are $C_5$- or $C_6$-cacloalkyl.

**14.** A protein adsorbed shapable electroconductive polymer film according to claim 5, wherein the substituents for cycloalkyl, phenyl and benzyl are one selected from the group of $C_1$-$C_4$-alkyl, $C_1$-$C_4$-alkoxy, F, Cl and Br.

**15.** A protein adsorbed shapable electroconductive polymer film according to claim 5, wherein $R_3$ and $R_4$ together as alkylene means $C_1$-$C_4$-alkylene.

**16.** A protein adsorbed shapable electroconductive polymer film according to claim 15, wherein $R_3$ and $R_4$ are $C_1$- or $C_2$-alkylene.

**17.** A protein adsorbed shapable electroconductive polymer film according to claim 15, wherein $R_3$ and $R_4$ as alkylene are methylene, ethylene, propylene or butylene.

**18.** A protein adsorbed shapable electroconductive polymer film according to claim 5, wherein the substituents for $R_3$ and $R_4$ as alkylene are one selected from the group of $C_1$-$C_6$-alkyl, $C_1$-$C_6$-alkoxy, cyclohexyl, phenyl and benzyl.

**19.** A protein adsorbed shapable electroconductive polymer film according to claim 5, wherein $R_3$ and $R_4$ are linear or branched $C_1$-$C_6$-alkyl or $R_3$ and $R_4$ together are $C_1$-$C_4$-alkylene which is unsubstituted or substituted by 1 or 2 $C_1$-$C_6$-alkyl groups or $C_1$-$C_6$-alkoxy groups.

**20.** A protein adsorbed shapable electroconductive polymer film according to claim 19, wherein $R_3$ and $R_4$

are linear or branched $C_1$-$C_4$-alkyl or $R_3$ and $R_4$ together are $C_1$- or $C_2$-alkylene.

**21.** A protein adsorbed shapable electroconductive polymer film according to claim 20, wherein $R_3$ and $R_4$ are $C_1$- or $C_2$-alkylene which is unsubstituted or substituted by $C_1$-$C_6$-alkyl or $C_1$-$C_6$-alkoxy.

**22.** A protein adsorbed shapable electroconductive polymer film according to claim 21, wherein $R_3$ and $R_4$ stand for a residue of formula $-CHR_a-CHR_b-$, wherein $R_a$ and $R_b$ are each independently of the other H or $C_1$-$C_6$-alkyl.

**23.** A protein adsorbed shapable electroconductive polymer film according to claim 22, wherein $R_a$ and $R_b$ are H, methyl or ethyl.

**24.** A protein adsorbed shapable electroconductive polymer film according to claim 5, wherein the structural unit of formula II corresponds to the formula

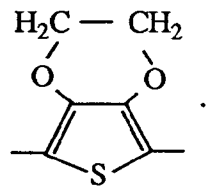

**25.** A protein adsorbed shapable electroconductive polymer film according to claim 1, wherein, per structural unit of the polyheteroaromatic compound, 0.1 to 0.9 structural units having sulfated alcoholic groups

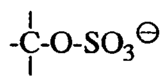

and/or sulfonatalkylated alcoholic groups

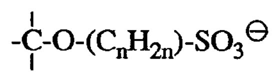

are contained.

**26.** A protein adsorbed shapable electroconductive polymer film according to claim 25, wherein, per structural unit of the polyheteroaromatic compound, 0.1 to 0.6 structural units having sulfated alcoholic groups

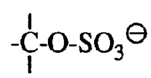

and/or sulfonatalkylated slcoholic groups

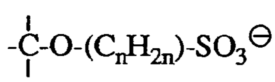

are contained.

**27.** A protein adsorbed shapable electroconductive polymer film according to claim 1, wherein the group $-(C_nH_{2n})-$ is linear or branched $C_3$-$C_8$-alkylene having 3 to 5 C-atomes in the carbon chain.

**28.** A protein adsorbed shapable electroconductive polymer film according to claim 27, wherein the group $-(C_nH_{2n})-$ is linear $C_3$-$C_5$-alkylene.

**29.** A protein adsorbed shapable electroconductive polymer film according to claim 28, wherein the group $-(C_nH_{2n})-$ is trimethylene or tatramethylene.

**30.** A protein adsorbed shapable electroconductive polymer film according to claim 1, wherein the thermoplastic polymer having sulfated alcoholic groups in salt form

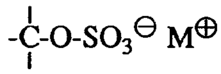

and/or sulfonatalkylated alcoholic groups in salt form

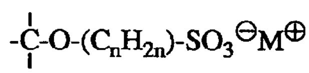

has a glass transition temperature of - 100 to 350°C measured by DSC-method, wherein $M^{\oplus}$ is an alkali metalic or ammonium cation.

**31.** A protein adsorbed shapable electroconductive polymer film according to claim 1, wherein the degree of polymerization of the thermoplastic polymer is 5 to 10000.

**32.** A protein adsorbed shapable electroconductive polymer film according to claim 1, wherein the alcoholic groups in the thermoplastic polymer are completely sulfated and/or sulfonatalkylated.

**33.** A protein adsorbed shapable electroconductive polymer film according to claim 1, wherein the ratio of free alcoholic groups to sulfated alcoholic groups

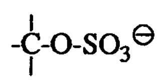

and/or sulfonatalkylated alcoholic groups

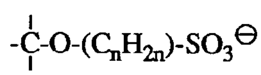

in the thermoplastic polymer is within the range of 50: 1 to 1 : 50.

**34.** A protein adsorbed shapable electroconductive polymer film according to claim 1, wherein the thermoplastic polymer contains 5 to 100 mol% of sulfated alcoholic groups

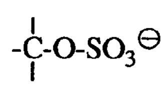

and/ or sulfonatalkylated alcoholic groups

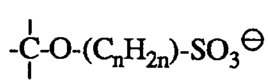 ,

wherein the remaining structural units are corresponding hydroxyl group-containing or hydroxyl group-free structural units.

**35.** A protein adsorbed shapable electroconductive polymer film according to claim 1, wherein the sulfated and/or sulfonatalkylated alcoholic groups are bound as secondary groups $>CH-O-SO_3^{\ominus}$ or $>CH-O-(C_nH_{2n})-SO_3^{\ominus}$ or as tertiary groups

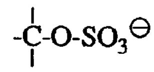

or

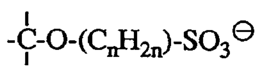

in the polymer backbone; or in side-chains of the polymer as primary groups $-CH_2-O-SO_3^{\ominus}$ or $-CH_2-O-(C_nH_{2n})-SO_3^{\ominus}$ in the terminal position, or as secondary groups $>CH-O-SO_3^{\ominus}$ or $>CH-O-(C_nH_{2n})-SO_3^{\ominus}$ or as tertiary groups

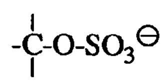

or

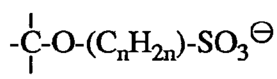

in a central position of the side chain.

**36.** A protein adsorbed shapable electroconductive polymer film according to claim 1, wherein the thermoplastic polymer is derived from at least one hydroxyl group-containing polymer selected from the group consisting of polyesters, polyesteramides, polyurethanes, polyamides, polycarbonates and polyimides from hydroxyl group-containing monomers; saponified homopolymers from vinylesters or -ethers; hydroxylated polyolefines as well as their copolymers with olefinic monomers; homopolyacrylates or homopolymethacrylates having hydroxy residues in the ester groups; polysiloxanes having hydroxyalkyl groups; reduced polyketones or comonomers thereof; polyesters obtained from glycidyl compounds and diols; polyvinyl phenol or copolymers of vinylphenol and olefinic comonomers; as well as copolymers of vinyl alcohol, or hydroxyalkyl-containing acrylates or methacrylates, or hydroxylated diolefines with ethylenically unsaturated comonomers.

**37.** A protein adsorbed shapable electroconductive polymer film according to claim 36, wherein the sulfated and/or sulfonatalkylated thermoplastic polymer is one selected from the group consisting of polyadducts of glycidyl compounds having on average more than one epoxide group and a diol; homo- or copolymers of hydroxyalkyl acrylates or -methacrylates; homo- or copolymers of butadiene, isoprene and chloroprene, whose double bonds are partially or completely hydroxylated; homo- or copolymers of vinylphenol; polyimides from hydroxylated ketotetracarboxylic acids; hydroxylated polysiloxanes; and polyesters, polyamides, polyurethanes or polyimides from $C_4$-$C_{12}$-alkenylene diols as well as -diamines which double bonds are hydroxylated.

**38.** A protein adsorbed shapable electroconductive polymer film according to claim 36, wherein the thermoplastic polymer is either sulfated or sulfonatalkylated.

**39.** A protein adsorbed shapable electroconductive polymer film according to claim 1, wherein the thermoplastic polymer is an at least partially sulfated and/ or sulfonatalkylated polyadduct from

a) a glycidyl compound having on average more than one epoxide group and

b) a diol,

the adduct containing the groups

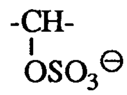

and/or

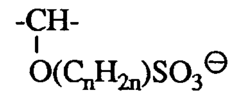

in the polymer chain, wherein the group $-(C_nH_{2n})-$ has the meaning given in claim 1.

**40.** A protein adsorbed shapable electroconductive polymer film according to claim 39, wherein the polyadduct contains

a) 100 to 5 mol% os identical or different structural units of formula III and/or IIIa

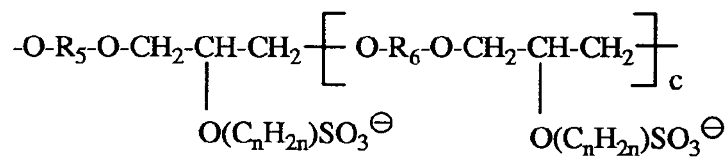

(III)

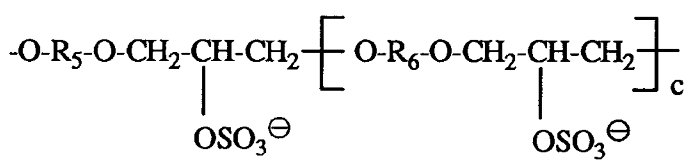

(IIIa)

and

b) 0 to 95 mol% of identical or different structural units of formula IV

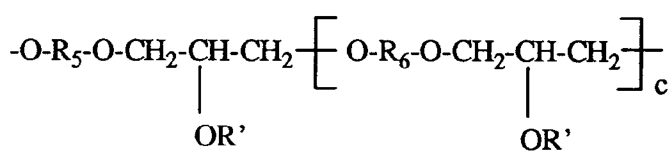

(IV)

based on the polyadduct, wherein $R_5$ and $R_6$ are each independently of the other the residue of a diol having aliphatic or aromatic diol groups, which residue is diminished by two hydroxyl groups, R' is H, $C_1$-$C_{20}$-alkyl, $C_1$-$C_{20}$-acyl or aminocarbonyl which is N-substituted by a $C_1$-$C_{20}$-hydrocarbon group, the group $-(C_nH_{2n})-$ is defined as in claim 1, and c is 0 or 1..

**41.** A protein adsorbed shapable electroconductive polymer film according to claim 40, wherein R' in formula IV is H.

**42.** A protein adsorbed shapable electroconductive polymer film according to claim 40, wherein $R_5$ and $R_6$ represent an identical residue.

**43.** A protein adsorbed shapable electroconductive polymer film according to claim 40, wherein $R_6$ and $R_6$ as residues having aliphatic diol groups are linear or branched $C_2$-$C_{12}$-alkylene, $C_3$-$C_8$-cycloalkylene, $C_1$-$C_4$-alkyl$C_5$-$C_8$-cycloalkylene, cyclohexylmethylene or cyclohexyldimethylene.

**44.** A protein adsorbed shapable electroconductive polymer film according to claim 40, wherein $R_5$ and $R_6$ represent each independently of the other a residue of formula V

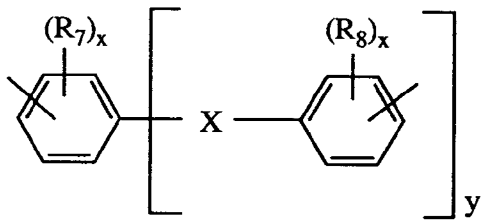

(V),

wherein X is a direct bond, $C_1$-$C_4$-alkylene, $C_2$-$C_{12}$-alkylidene, $C_5$-$C_8$-cycloalkylidene, -O-, S, SO-, $-SO_2-$, -CO-, $-CO_2-$, -N($C_1$-$C_4$-alkyl)- or $-Si(CH_3)_2-$, and $R_7$ and $R_8$ are each independently of the other H, halogen, $C_1$-$C_4$-alkyl or $C_1$-$C_4$-alkoxy, and x is 1 or 2 and y is 0 or 1.

**45.** A protein adsorbed shapable electroconductive polymer film according to claim 44, wherein X is a direct bond, methylene, ethylene, $C_2$-$C_6$-alkylidene, cyclohexylidene or cyclopentylidene, -O- or -S-.

**46.** A protein adsorbed shapable electroconductive polymer film according to claim 44, wherein $R_7$ and $R_6$ are H and y is 1.

**47.** A protein adsorbed shapable electroconductive polymer film according to claim 40, wherein $R_5$ and $R_6$ represent the residue

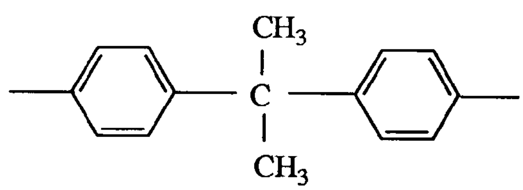

.

**48.** A protein adsorbed shapable electrocondcutive polymer film according to claim 1, wherein the thermoplastic polymer is an at least partially sulfated and/or sulfonatalkylated polyvinyl alcohol or sulfated and/or sulfonatalkylated polyvinyl alcohol having the groups

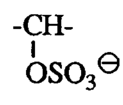

and/or

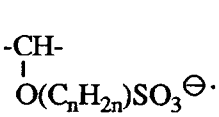

.

**49.** A protein adsorbed shapable electroconductive polymer film according to claim 48, wherein the thermoplastic polymer is a sulfated and/or sulfonatalkylated polyvinyl alcohol-copolymer.

**50.** A protein adsorbed shapable electroconductive polymer film according to claim 49, wherein the polyvinyl alcohol-copolymer contains

a) 90 to 5 mol% of structural units of formula VI and/or VIa

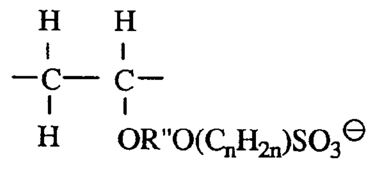

(VI)

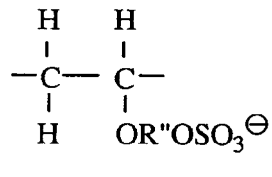

(VIa)

and

b) 10 to 95 mol% of identical or different structural units of formula VII

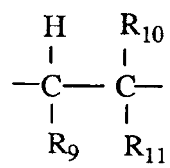

(VII),

wherein $R_9$ stands for H, $C_1$-$C_6$-alkyl, -$COOR_{12}$ or -$COO^{\ominus}$, $R_{10}$ means H, F, Cl, CN or $C_1$-$C_6$-alkyl, $R_{11}$ represents H, OH, R"OH, F, Cl, CN, $R_{12}$-O-, $C_1$-$C_{12}$-alkyl, -$COO^{\ominus}$, -$COOR_{12}$, -OCO-$R_{12}$, methylphenyl or phenyl, $R_{12}$ represents $C_1$-$C_{18}$-alkyl, $C_5$ $C_7$-cycloalkyl, ($C_1$-$C_{12}$-alkyl)-$C_5$-$C_7$-cycloalkyl, phenyl, ($C_1$-$C_{12}$-alkyl)phenyl, benzyl or ($C_1$-$C_{12}$-alkyl)benzyl, R" is linear or branched $C_2$-$C_{18}$-alkylene or poly($C_2$-$C_6$-oxaalkylene) having 2 to 6 oxaalkylene units, and the group -$C_nH_{2n}$- is defined as in claim 1.

**51.** A protain adsorbed shapable electroconductive polymer film according to claim 50, wherein the copolymer contains 60 to 10 mol% of structural units of formula V and 40 to 90 mol% of structural units of formula VI.

**52.** A protein adsorbed shapable electroconductive polymer film according to claim 50, wherein R" as alkylene contains 2 to 12 C-atoms, and R" as poly(oxaalkyl) contains 2 to 4 oxaalkyl units having 2 to 4 C-atoms in the alkyl group.

**53.** A protein adsorbed shapable electroconductive polymer film according to claim 50, wherein $R_9$ is H, $R_{10}$ is H, F, Cl, methyl or ethyl, and $R_{11}$ is H, OH, F, Cl, CN, $C_1$-$C_6$-alkyl, $C_1$-$C_6$-alkoxy, -COO-$C_1$-$C_6$-alkyl,

-OOC-$C_1$-$C_6$-alkyl or phenyl.

**54.** A protein adsorbed shapable electroconductive polymer film according to claim 53, wherein in formula V $R_9$ means H, $R_{10}$ is H or methyl, and $R_{11}$ is H, OH, CN, methyl, $OCH_3$ or -$COOCH_3$.

**55.** A protein adsorbed shapable electroconductive polymer film according to claim 50, wherein 20 to 60 mol% of structural units of formula VI and/or VIa, 50 to 40 mol% of structural units of formula -$CH_2$-$CH_2$- and 30 to 0 mol% of structural units of formula -$CH_2$-CH(OH)- are contained, wherein the sum of the mol% is 100 percent.

**56.** A protein adsorbed shapable electroconductive polymer film accordeing to claim 1, wherein the thermoplastic polymer is a sulfated and/or sulfonatalkylated polymer of hydroxylated polybutadiene, chloroprene or polyisoprene.

**57.** A protein adsorbed shapable electroconductive polymer film according to claim 56, wherein the thermoplastic polymer contains 5 to 100 mol% of structural units of formula VIII and/or VIIIa

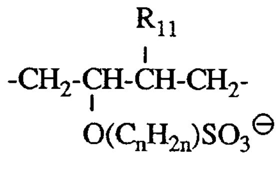

(VIII)

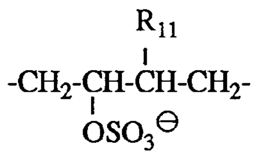

(VIIIa)

and 95 to 0 mol% of structural units of formula IX and/or IXa

$$-CH_2-CH=CH-CH_2- \qquad (IX)$$

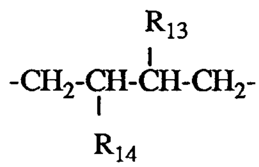

(IXa),

wherein $R_{13}$ and $R_{14}$ are each independently of the other H, OH or Cl and the group $-(C_nH_{2n})-$ has the meaning given in claim 1.

**58.** A protein adsorbed shapable electroconductive polymer film according to claim 57, wherein 10 to 100 mol% of structural units of formula VIII and/or VIIIa and 90 to 0 mol% of structural units of formula IX and/or IXa are contained.

**59.** A protein adsorbed shapable electroconductive polymer film according to claim 58, wherein 20 to 60 mol% of structural units of formula VIII and/or VIIIa and 80 to 40 mol% of structural units of formula IX and/or IXa are contained.

**60.** A protein adsorbed shapable electroconductive polymer film according to claim 57, wherein $R_{13}$ is Cl or H and $R_{14}$ is H.

**61.** A protein adsorbed shapable electroconductive polymer film according to claim 1, wherein the film-forming thermoplastic polymer contains

a) 100 to 50 mol% of identical or different structural units of formula III and/or IIIa

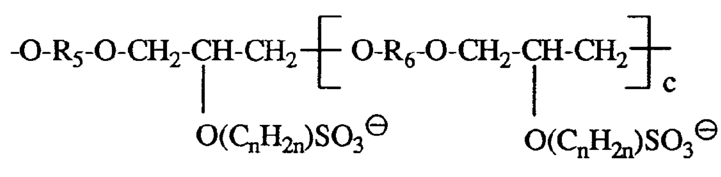

(III)

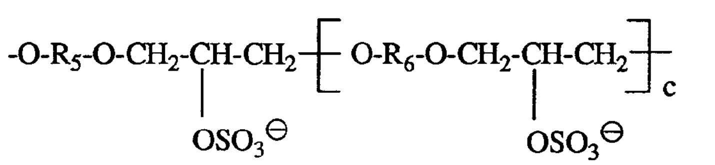

(IIIa)

and

b) 0 to 50 mol% of identical or diffrerent structural units of formula IV

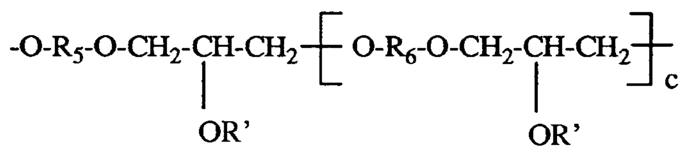

(IV)

based on the polyadduct, wherein $R_5$ and $R_6$ are each independently of the other a residue of a diol having aliphatic or aromatic diol groups, the residue being diminished by two hydroxy groups, R' is H, $C_1$-$C_{20}$-alkyl, $C_1$-$C_{20}$-acyl or aminocarbonyl which is N-substituted by a $C_1$-$C_{20}$-hydrocarbon group, the group $-(C_nH_{2n})-$ is defined as in claim 1, and c is 0 or 1.

**62.** A protein adsorbed shapable electroconductive polymer film according to claim 61, wherein the polymer contains 60 to 100 mol% of structural units of formula III and/or IIIa and 40 to 0 mol% of structural units of firmula IV.

**63.** A protein adsorbed shapable electroconductive polymer film according to claim 62, wherein the polymer contains 60 to 90 mol% of structural units of formula III and/or IIIa and 40 to 10 mol% of structural units of formula IV.

**64.** A protein adsorbed shapable electroconductive polymer film according to claim 61, wherein R' is H.

**65.** A protein adsorbed shapable electroconductive polymer film according to claim 61, wherein polypyrrole in oxidized, polycationic form having structural units of formula I'

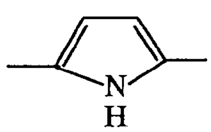

(I')

or polythiophene in oxidized, cationic form having structural units of formula II

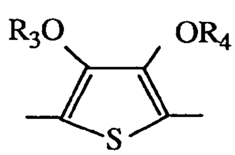

(II)

is contained, wherein $R_3$ and $R_4$ are each independently of the other linear or branched $C_1$-$C_6$-alkyl or $R_3$ and $R_4$ altogether repesent methylene or the group $-CHR_a-CHR_b-$, and $R_a$ and $R_b$ are each independently of the other H, methyl or ethyl.

**66.** A protein adsorbed shapable electroconductive polymer film according to claim 65, wherein $R_a$ and $R_b$ are respectively H.

**67.** A protein adsorbed shapable electroconductive polymer film according to claim 61, wherein $R_5$ and $R_6$ are each independently of the other a residue of formula V

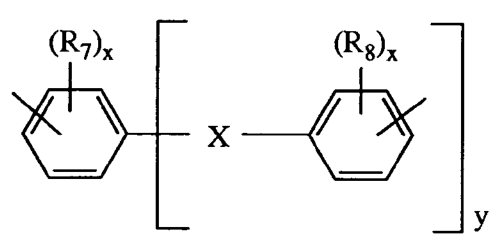

(V),

wherein X is a direct bond, $C_1$-$C_4$-alkylene, $C_2$-$C_{12}$-alkylidene, $C_5$-$C_8$-cycloallcylidene, -O-, -S-, -SO-, -CO-, $-CO_2-$, $-N(C_1$-$C_4$-alkyl)- or $-Si(CH_3)_2-$, $R_7$ and $R_8$ are each independently of the other H, halogen, $C_1$-$C_4$-alkyl or $C_1$-$C_4$-alkoxy, and x is 1 or 2 and y is 0 or 1.

**68.** A protein adsorbed shapable electroconductive polymer film according to claim 67, wherein X is a direct bond, methylene, ethylene, $C_2$-$C_6$-alkylidene, cycloalkylidene or cyclopentylidene, -O-or -S-.

**69.** A protein adsorbed shapable electroconductive polymer film according to claim 67, wherein $R_7$ and $R_8$ are H or methyl and y is 1.

**70.** A protein adsorbed shapable electroconductive polymer film according to claim 67, wherein $R_5$ and $R_8$ mean the residue

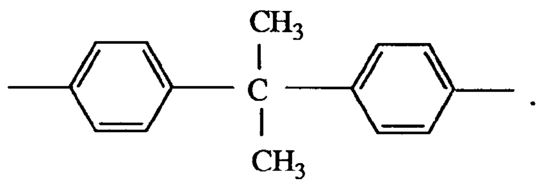

**71.** A protein adsorbed shapable electrocondcutive polymer film according to claim 65, wherein the structural units of polythiophene are represented by formula

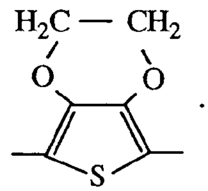

**72.** A protein adsorbed shapable electroconductive polymer film according to claim 1, wherein the protein adsorbed on the film is one selected from the group consisting of glucoseoxidase, D-amino acid oxidase, L-amino acid oxidase, choline oxidase, xanthine oxidase, sarcosine oxidase, L-lactate oxidase, pyruvate oxidase, ascorbate oxidase, cholesterol oxidase, fructose dehydrogenase, sorbitol dehydrogenase, alcohol dehydrogenase, lactate dehydrogenase, malate dehydrogenase, glutamate dehydrogenase, glycerol dehydrogenase, 11B hydroxysteroid dehydrogenase, nitrate reductase, oestradiol 17B dehydrogenase, amino acids dehydrogenase, $\alpha$-chymotripsin, tripsin, papain, pepsin, subtilisin, thermoase, thermolysis, lipase, and any antigen and antibody.

**73.** A protein adsorbed shapable electroconductive polymer film according to claim 1, wherein the film is plasma etched prior to protein adsorption.

**74.** A protein adsorbed shapable electroconductive polymer film according to claim 73, wherein the plasma is air plasma, nitrogen plasma, oxygen plasma, ammonium plasma or argon plasma.

**75.** A protein adsorbed shapable electroconductive polymer film according to claim 1, wherein 0.1 to 50 $\mu g/cm^2$ protein are adsorbed.

**76.** A protein adsorbed shapable electroconductive polymer film according to claim 75, wherein 0.2 to 10 $\mu g/cm^2$ protein are adsorbed

**77.** A process for the preparation of a protein adsorbed shapable electroconductive polymer film according to claim 1, wherein a film of component 1) of claim 1 is contacted with an aqueous protein solution.

**78.** A process according to claim 77, wherein the adsorption is potential-aided by applying voltage.

**79.** Use of the protein adsorbed shapable electroconductive polymer film according to claim 1 as biosensor material, bioreactor material or immunosensor material.